# EUROPÄISCHE PATENTSCHRIFT

(11) **EP 1 019 719 B1**
(45) Veröffentlichungstag und Bekanntmachung des Hinweises auf die Patenterteilung: **23.03.2005**
(21) Anmeldenummer: 99939437.2
(22) Anmeldetag: 03.08.1999
(51) Int. Cl.: G01N 33/50

(54) **VERFAHREN ZUR BESTIMMUNG DES ZUSTANDES VON KERATIN ENTHALTENDEN MATERIALIEN**
METHOD FOR DETERMINING THE STATE OF SUBSTANCES CONTAINING KERATIN AND SUITABLE DEVICES AND MEANS THEREFOR
PROCEDE PERMETTANT DE DETERMINER L'ETAT DE MATIERES CONTENANT DE LA KERATINE, AINSI QUE DISPOSITIF ET MOYENS ASSOCIES

(30) Priorität: 04.08.1998 DE 19835055
(43) Veröffentlichungstag der Anmeldung: 19.07.2000
(73) Patentinhaber: Wella Aktiengesellschaft, 64274 Darmstadt (DE)
(72) Erfinder: SCHWAN-JONCZYK, Annette, D-64295 Darmstadt (DE); HAALCK, Lutz, D-48149 Münster (DE); ÜNSAL, Ceylan, D-40670 Meerbusch (DE); FELDBRÜGGE, Rainer, D-48143 Münster (DE)
(86) Internationale Anmeldenummer: PCT/EP1999/005568
(87) Internationale Veröffentlichungsnummer: WO 2000/008465

(56) Entgegenhaltungen:
- EP-A- 0 458 594
- EP-A- 0 458 644
- EP-A- 0 702 232
- US-A- 4 304 488
- US-A- 5 443 961
- US-A- 5 580 785
- PATENT ABSTRACTS OF JAPAN vol. 1996, no. 6, 28. Juni 1996 (1996-06-28) & JP 08 043375 A (POLA CHEM IND), 16. Februar 1996 (1996-02-16)

## Beschreibung

Die vorliegende Erfindung betrifft ein Verfahren zur Feststellung und Bestimmung von Zuständen Keratin enthaltender Hautanhangsgebilden, insbesondere von Zuständen des Haares, mit bestehenden strukturellen Schäden, die durch äußere Einflüsse wie Umwelt, Bewetterung, natürliche Alterung, physikalische oder chemische Noxen oder kosmetische Behandlungen verursacht werden, sowie hierfür geeignete Mittel und Vorrichtungen und die Verwendung derselben.

Keratin enthaltende Hautanhangsgebilden, welche Haare, Wolle, Federn, umfassen, unterliegen einer Vielzahl von natürlichen und nicht-natürlichen, physikalischen und chemischen Einflüssen. Hierzu zählen energiereiche Strahlungen (zum Beispiel UV-Licht), Wetterbedingungen bzw. Bewetterung (zum Beispiel Temperatur, Luftfeuchtigkeit oder die Luftzusammensetzung), kosmetische Behandlungen, wie zum Beispiel Bleichen der Haare, Dauerwellbehandlungen, Färbungen oder Lackierungen. Derartige Einflüsse äußern sich in einer unterschiedlich ausgeprägten strukturellen Veränderung dieser Hautanhangsgebilden wodurch sich ihre chemischen und physikalischen Eigenschaften verändern können. Im Falle von beispielsweise Haaren kann es als Folge davon häufig zu Verlusten an Glanz, Geschmeidigkeit, Halt oder Kämmbarkeit kommen. Zusätzlich nimmt die Brüchigkeit und Spliss von Haaren zu. Der Grad der Schädigung steigt mit Häufigkeit und Expositionsdauer der einwirkenden Parameter.

Für eine wirkungsvolle Anwendung von Pflege-, Konditionierungs- oder Restrukturierungsmitteln an Haaren ist es notwendig, nicht nur den jeweiligen Haarzustand sicher zu erkennen. Insbesondere aber die richtige der jeweiligen Haarstruktur angepaßte Auswahl der Dauerwell-, Färbe- und Bleichmittel erfordert eine sichere Bestimmung des Schädigungsgrades der Haare. Die Erfassung von Haarzuständen kann einerseits optisch oder taktil durch subjektive Beurteilung von beispielsweise Glanz, Brüchigkeit, Rauigkeit oder Auftreten von Spliss, andererseits durch objektive Meßverfahren erfolgen.

Die Methode der subjektiven Beurteilung von Haarzuständen bzw. von Haarschädigungen ist schnell und einfach. Jedoch führt die Erfassung anfänglicher und fortgeschrittener struktureller Veränderungen (Haarschädigungen) durch subjektive Parameter zu stark fehlerhaften oder nicht verwertbaren Resultaten, entweder durch fehlende Erfahrung bei der Einschätzung des Haarzustandes bzw. der Schädigung oder durch Maskierung einer Schädigung durch Haarpflegemittel.

Für eine objektive Untersuchung von Keratin enthaltenden Hautanhangsgebilden zur Feststellung eventuell vorliegender Schädigungen sind eine Anzahl Verfahren bekannt. Beispielsweise die Bestimmung des Cysteinsäuregehalts mit Hilfe der Hochdruckflüssigkeits-Chromatographie (HPLC). Dieses Verfahren hat den Nachteil, daß umfangreiche Laboruntersuchungen notwendig sind, die zeit- und kostenaufwendig sind. Ein solches Verfahren ist daher für die tägliche Anwendung, insbesondere für den Friseur oder Kosmetiker, nicht praktikabel.
Es ist ferner bekannt, Haarschädigungen durch Verwendung von anionischen Farbstoffen zu bestimmen (beispielsweise EP-A 0 702 232). Dieses Verfahren liefert jedoch nur unzureichende Informationen über den strukturellen Zustand von Haaren. Weitere bekannte Verfahren bestehen darin, den Ausdehnungsfaktor des betreffenden Haares zu bestimmen oder den Grad der Kupferaufnahme von geschädigtem Haar zu messen. Derartige Verfahren sind beschrieben in den U.S.-Patenten 5,461,925 und 4,665,741. Diese Methoden sind zu unspezifisch und können falsch-positive oder falsch-negative Resultate liefern. Außerdem sind diese Verfahren zeitraubend und apparativ aufwendig.

Da einerseits eine zuverlässige Feststellung und Bewertung einer bestehenden Schädigung von Keratin enthaltenden Hautanhangsgebilden unablässig ist für eine effektive und folgerichtige kosmetische Behandlung und andererseits kein praktikables Verfahren für einen für diesen Zweck geeigneten Schnelltest, dafür geeignete Vorrichtungen oder Mittel für die Durchführung eines solchen Verfahrens, bekannt sind, besteht ein großes Bedürfnis für einen Schnelltest und für die dafür geeigneten Vorrichtungen und Mittel.

Aufgabe der vorliegenden Erfindung ist es, die Nachteile des Standes der Technik zu beseitigen und ein zuverlässiges Verfahren zur Feststellung und Bewertung von schadhaften Zuständen von Keratin enthaltenden Hautanhangsgebilden insbesondere von Haaren, sowie die für dieses Verfahren notwendigen Vorrichtungen und Mittel bereitzustellen.

Die Aufgabe wurde im Umfang der vorliegenden Ansprüche gelöst.

Überraschenderweise konnte nämlich festgestellt werden, daß Keratin enthaltende Hautanhangsgebilde, wie zum Beispiel Haare, unterschiedlichen Schädigungsgrades auf enzymatische und/oder chemische Behandlung in wäßriger Lösung unterschiedlich reagieren und daß die Flüssigkeitsprobe in Abhängigkeit des Schädigungsgrades der Keratin enthaltenden Hautanhangsgebilde unterschiedlich starke Trübungen zeigt.

Demzufolge ist ein Aspekt der vorliegenden Erfindung ein Verfahren zur Feststellung und Bestimmung des Zustandes von Keratin enthaltenden Hautanhangsgebilden, welches darin besteht, daß eine Probe von Keratin enthaltende Hautanhangsgebilde, beispielsweise eine Haarprobe, in einer wäßrigen Lösung einer enzymatischen und/oder chemischen Behandlung unterworfen wird, und anschließend der Zustand der Haare anhand der Trübung der erhaltenen wäßrigen Lösung untersucht, ausgewertet und bestimmt wird. Die Bestimmung des Zustandes der Haare kann leicht mittels eines Vergleichswerts (Nullwert oder Standardwert) erfolgen. Ein Vergleichswert kann erhalten werden, in dem eine gleiche Menge an gesundem Haar unter identischen Bedingungen wie die Haarprobe einer enzymatischen und/oder chemischen Behandlung ausgesetzt und die Trübung der erhaltenen Flüssigkeitsprobe festgestellt wird.

Unter dem Begriff "Bestimmung des Zustandes von Keratin enthaltenden Hautanhangsgebilden" ist insbesondere die Bestimmung von möglichen oder zu erwartenden Schädigungen der betreffenden Materialien zu verstehen. Hinsichtlich der nach der enzymatischen und/oder chemischen Behandlung einer Probe Keratin enthaltenden Hautanhangsgebilden, beispielsweise einer Haarprobe, erhaltenen wäßrigen Lösung wird diese im weiteren als "Flüssigkeitsprobe" bezeichnet

Das erfindungsgemäße Verfahren vereint eine Anzahl von Vorteilen. Es wird ein Schnelltest zur Bestimmung des Zustandes von Keratin enthaltenden Hautanhangsgebilden bereitgestellt, der innerhalb kürzester Zeit verläßliche Aussagen über den jeweiligen Zustand Keratin enthaltender Hautanhangsgebilde erlaubt und eine Diagnose hierüber erstellbar macht. Es werden reproduzierbare Ergebnisse geliefert, die den Anwender in die Lage versetzt, Veränderungen des Zustandes Keratin enthaltender Hautanhangsgebilde uber einen längeren Zeitraum nach objektiven Kriterien zu verfolgen und hierüber eine Datei anzulegen.
Ganz besonders vorteilhaft kann das erfindungsgemäße Verfahren auf dem Gebiet der Haarpflege bzw. Haarkosmetik angewendet werden. Damit eröffnen sich aber auch Möglichkeiten einer flexiblen und individuellen, auf eine kausale Haarpflege zielende kosmetische Haarbehandlung für den Anwender, insbesondere den Friseur. Das Verfahren gestattet eine optische oder visuelle Untersuchung der Flüssigkeitsprobe und ist daher einfach durchzuführen. Die hierfür benötigten Mittel und Vorrichtungen sind leicht zu handhaben. Durch die hohe Reproduzierbarkeit und die differenzierte Untersuchungsmöglichkeit des Zustandes bzw. des Schädigungsgrades der Keratin enthaltenden Hautanhangsgebilde verbunden mit der leichten Handhabung stellt das erfindungsgemäße Verfahren und die dafür geeigneten erfindungsgemäßen Vorrichtungen und Mittel eine ideale Lösung zur schnellen Beurteilung insbesondere der Haarqualität dar.

Für das erfindungsgemäße Verfahren können alle enzymatischen und/oder chemischen Verfahren angewendet werden, soweit diese eine proteolytische oder hydrolytische Zersetzung von Haaren hervorrufen. Demzufolge umfaßt das erfindungsgemäße Verfahren eine enzymatische und/oder chemische Behandlung einer Probe von Keratin enthaltenden Hautanhangsgebilden (zum Beispiel einer Haarprobe) auf Basis einer proteolytischen oder hydrolytischen Reaktion. Hierfür eignen sich rein enzymatische Behandlungen, rein chemische Behandlungen oder kombinierte, enzymatisch/chemische Behandlungen der zu untersuchenden Materialien.

Betreffend enzymatische Verfahren kommen alle hierfür geeigneten und bekannten Enzyme aus der Gruppe der Proteasen und Proteinasen (Exo- und Endopeptidasen bzw. Exo- und Endoproteasen und Gemischen davon) in Betracht, die in der Lage sind, einen Abbau von keratinhaltigem Material zu katalysieren bzw. Keratinase-Aktivität aufweisen, wobei mindestens ein Enzym für die enzymatische (oder auch enzymatisch/chemische) Behandlung in der betreffenden wäßrigen Lösung vorhanden ist. Die Enzyme können einzeln oder als Gemisch vorliegen. Hierzu zählen beispielsweise Papain, Pronase E, Proteinnase K, Subtilisin oder Trypsin, sowie Keratinasen, beispielsweise aus *Fervidobacterium pennavorans* (FRIEDRICH, A. B. und ANTRANIKIAN, G., Appl. Environ. Microbiol. 62:2875, 1996), aus *Streptomyces pactum*, DSM 40530, (BOECKLE, B. et al., Appl. Environ. Microbiol. 61:3705, 1995), aus *Candida pulcherima* KSY 188-5 (GOTOCH, T. et al., Biosci. Biotechnol. Biochem. 59:367, 1995) oder aus *Bacillus licheniformis* PWD-1 (LIN, X. Appl. Environ. Microbiol. 61(4):1469-1474, 1995, CHENG, S.W., Biosci. Biotechnol. Biochem. 59(12):2239-2243, 1995). Die Verwendung von speziellen Keratinasen kann von Vorteil sein, da sie den proteolytischen Abbau von Keratin enthaltenden Hautanhangsgebilden beschleunigen können. So ist beispielsweise von der Keratinase *Fervidobacterium pennavorans* bekannt, daß diese Hühnerfedem schnell auflösen können (FRIEDRICH, A. B. und ANTRANIKIAN, G., Appl. Environ. Microbiol. 62:2875, 1996).
Die für das erfindungsgemäße Verfahren geeigneten Enzyme sind kommerziell erhältlich, beispielsweise Papain (isolierbar aus *Carica papaya*) von Boehringer Mannheim, Pronase E (isolierbar aus *Streptomyces griseus*) von Sigma (Deisenhofen) oder Proteinase K (isolierbar aus *Tritirachium album*) von Merck (Darmstadt).

Hinsichtlich rein chemischer Verfahren zur Zersetzung von Keratin enthaltenden Hautanhangsgebilden kommen solche organischen und/oder anorganischen Reagenzien oder Lösungen davon in Frage, die bevorzugt Keratin zu hydrolysieren vermögen und demzufolge keratolytisch wirksam sind und/oder reduzierende Eigenschaften aufweisen. Hierfür geeignete organische oder anorganische Reagenzien umfassen Reduktionsmittel, welche sowohl unter basischen als auch unter sauren Bedingungen zur Anwendung kommen können. Die betreffenden organischen und anorganischen Reagenzien können einzeln oder als Gemisch in der entsprechenden wäßrigen Lösung vorliegen. In Betracht kommen beispielsweise partielle wäßrige reduktive Hydrolysen mit beispielsweise Harnstoff-Bisulfit-Lösungen (zum Beispiel eine Lösung mit einem Gehalt an Harnstoff und Natriummetabisulfit) gemäß IWTO-Spezifikation (IWTO 11-62 (D), Neue Ausgabe 1966), partielle alkalisch-wäßrige Hydrolysen mit alkalischen Lösungen wie zum Beispiel Natriumhydroxidlösungen gemäß IWTO-Spezifikation (IWTO 4-60, neue Ausgabe 1996), partielle alkoholischreduktive Hydrolysen mit Tributylphosphin und Natriumjodid-Lösungen gemäß KILPATRIK, D. J. et al., Text. Res. J. 40:25, 1970, partielle sauer-basische Hydrolysen mit Perameisensäure/Ammoniak-Lösungen, partielle basisch-reduktive Hydrolysen mit Thioglykolsäure/Harnstoff-Lösungen oder partiell sauer-reduktive Hydrolysen mit Thioglykolsäure oder Cystein/Eisessig-Lösungen, sowie Cystein, Natrium-metabisulfit, Natriumsulfit, Dithiothreitol (DTT), Dithioerythrol (DTE).
Insofern umfaßt die vorliegende Erfindung auch ein Verfahren, welches dadurch gekennzeichnet ist, daß eine Probe Keratin enthaltender Hautanhangsgebilde, insbesondere eine Haarprobe, in einer wäßrigen Lösung einer chemischen Behandlung unterworfen wird und daß die dieser Behandlung zugrundeliegende proteolytische oder hydrolytische Reaktion mit Hilfe von mindestens einem keratolytisch wirksamen und/oder reduzierenden organischen und/oder anorganischen Reagenz erfolgt.

Unter kombinierten enzymatisch/chemischen Behandlungen der zu untersuchenden Keratin enthaltenden Hautanhangsgebilde sind solche zu verstehen, bei denen die gewünschte Hydrolyse bzw. Proteolyse der Keratin enthaltenden Hantanhangsgebilde unter Beteiligung von sowohl Enzymen als auch rein chemischen Stoffen erfolgt. In diesem Fall enthält die Flüssigkeitsprobe, in der die Materialprobe (zum Beispiel Haarprobe) der enzymatisch/chemischen Behandlung unterworfen wird, mindestens eines der oben genannten Enzyme zusammen mit mindestens einem keratolytisch wirksamen und/oder reduzierenden organischen und/oder anorganischen Reagenz. Hierfür eignen sich vorteilhafterweise Aminosäuren oder Sulfite (beispielsweise Cystein, Natriummetabisulfit, Natriumsulfit, Dithiothreitol (DTT), Dithioerythrol (DTE)). Eine solche kombinierte enzymatisch/chemische Behandlung ist ein bevorzugter Gegenstand der vorliegenden Erfindung.
Demgemäß liegt ein bevorzugtes erfindungsgemäßes Verfahren darin, daß eine Probe von Keratin enthaltenden Hautanhangsgebilden, insbesondere eine Haarprobe, in einer wäßrigen Lösung einer enzymatischen und chemischen Behandlung unterworfen wird und daß die dieser Behandlung zugrundeliegende proteolytische oder hydrolytische Reaktion mit Hilfe mindestens eines Enzyms, wie oben definiert, in Kombination mit mindestens einem keratolytisch wirksamen und/oder reduzierenden organischen und/oder anorganischen Reagenz, wie oben definiert, erfolgt.
Das Enzym kann zuerst und anschließend das organische und/oder anorganische Reagenz in die wäßrige Reaktionslösung eingebracht werden, oder das Enzym und das Reagenz kann gleichzeitig verwendet werden oder das organische und/oder anorganische Reagenz wird zuerst vorgelegt und anschließend das Enzym in die Reaktionslösung gegeben. Es kann zweckmäßig sein, das Verfahren derartig durchzuführen, daß das organische und/oder anorganische Reagenz zuerst in die wäßrige Reaktionslösung eingebracht und anschließend das Enzym hinzugegeben wird.

Die erfindungsgemäße Untersuchung und Auswertung der Flüssigkeitsprobe kann während und/oder nach der enzymatischen und/oder chemischen Behandlung erfolgen. Insofern wird erfindungsgemäß ein Verfahren umfaßt, wonach Gegenstand die Untersuchung und Auswertung der Flüssigkeitsprobe während und/oder nach der enzymatischen und/oder chemischen Behandlung erfolgen kann.

Die Mengen der erfindungsgemäß zu verwendenden Enzyme und organischen/anorganischen Reagenzien hängt natürlich ab von der Wahl der Enzyme, der Reagenzien, der Menge und Art der Probe der Keratin enthaltenden Hautanhangsgebilde und den Bedingungen, unter denen die enzymatisch und/oder chemische Behandlung der Probe erfolgt (insbesondere Temperatur und pH-Wert). Selbstverständlich ist hierbei auch zu berücksichtigen, daß Enzyme verschiedene Temperatur-Optima aufweisen können und daß auch der pH-Wert auf die enzymatische Aktivität Einfluß nimmt Die optimalen Bedingungen, unter denen Proteasen, Proteinasen oder Peptidasen ihre höchste proteolytische Aktivität entfalten, sind dem Fachmann bekannt. Sie können auch der vielfältigen Literatur entnommen oder über die verschiedenen Anbieter von Enzymen erhalten werden. Darüber hinaus hängen die Art der zu verwendenden Enzyme und der weiteren chemischen Reagenzien sowie deren eingesetzte Mengen davon ab, innerhalb welcher Zeit die Keratin enthaltenden Hautanhangsgebilden proteolytisch zersetzt werden sollen, um gewünschte Trübungen der zu untersuchenden Flüssigkeitsprobe zu erhalten. Die Bedingungen, unter denen eine gewünschte Trübung mit bestimmten Mengen an Enzymen und/oder chemischen Reagenzien unter bestimmten Bedingungen (Temperatur und pH-Wert) mit einer bestimmten Menge einer Haarprobe erhalten wird, kann selbstverständlich vom Fachmann leicht über vergleichende Versuche ermittelt und an seine Bedürfnisse angepaßt werden.

Im allgemeinen kann davon ausgegangen werden, daß die erfindungsgemäß zu verwendenden Enzyme in einer Menge (spezifische Aktivität) zwischen 1,0 und 500 U/ml, insbesondere zwischen 5,0 und 250 U/ml, ganz besonders zwischen 5,0 und 35,0 U/ml eingesetzt werden können. Es konnte festgestellt werden, daß eine spezifische Aktivität an Enzymen, insbesondere in Verbindung mit einem hydrolytisch wirkenden, keratolytisch wirksamen und/oder reduzierenden chemischen Reagenz, im Bereich zwischen 5,0 und 35,0 U/ml ausreicht, um Trübungen innerhalb von 5 bis 30 Minuten zu erreichen, die eine zuverlässige und reproduzierbare Bestimmung von Zuständen bzw. Schädigungen von Keratin enthaltenden Hautanhangsgebilden, insbesondere von Haarschädigungen, durchführen lassen.

Betreffend die Menge an erfindungsgemäß zu verwendenden keratolytisch wirksamen und/oder reduzierenden chemischen Reagenzien lassen sich aufgrund der Verschiedenartigkeit der in Betracht kommenden Reagenzien keine festen Zahlen angeben. Durch einfaches Ausprobieren läßt sich jedoch die gewünschte Menge für die Proteolyse oder Hydrolyse einer Probe an Keratin enthaltenden Hautanhangsgebilden und zur Erreichung einer für die Bestimmung eines Zustandes oder einer Schädigung des Materials geeigneten Trübung leicht ermitteln. Es hat sich jedoch als zweckmäßig erwiesen, für die betreffenden anorganischen chemischen Reagenzien Mengen zwischen 0,5 und 50,0 mg/ml, insbesondere zwischen 1,0 und 25,0 mg/ml, ganz besonders zwischen 2,0 und 15,0 mg/ml zu verwenden, vorteilhafterweise in Kombination mit einem entsprechenden Enzym. Betreffend organische chemische Reagenzien, wie beispielsweise Cystein (insbesondere L-Cystein), kommen Mengen zwischen 0,1 mg/ml und 50 mg/ml in Betracht, insbesondere zwischen 0,2 und 30 mg/ml, ganz besonders zwischen 0,5 und 25 mg/ml. Höhere Cystein-Konzentrationen in der wäßrigen Lösung (Reaktionslösung) sind insbesondere dann zu wählen, wenn Cystein als einziges chemisches Reagenz vorhanden ist, vorzugsweise in Kombination mit einem erfindungsgemäß geeigneten Enzym. Niedrige Cystein Mengen können vorteilhafterweise dann gewählt werden, wenn mindestens ein weiteres anorganisches keratolytisch wirksames und/oder reduzierendes Reagenz, beispielsweise ein oder mehrere Sulfite, zusammen mit Cystein vorhanden ist, insbesondere in Kombination mit einem erfindungsgemäß zu verwendenden Enzym.

Die Anwesenheit von mindestens einem anorganischen chemischen Reagenz (beispielsweise einem oder mehrerer Sulfite) zusammen mit einem organischen chemischen Reagenz (beispielsweise Cystein) muß, insbesondere in Kombination mit einem geeigneten Enzym, nicht zwingend notwendig sein für einen optimalen proteolytischen Abbau einer Probe Keratin enthaltender Hautanhangsgebilde. Es hat sich herausgestellt, daß sich die Reaktionsgeschwindigkeit bei geringer Cysteinkonzentration im Bereich von 0,1 bis 2,0 mg/ml durch alleinige Anwesenheit mindestens eines anorganischen chemischen Reagenzes, beispielsweise Sulfiten (zum Beispiel in einer Gesamtmenge von 10 mg an Natriummetabisulfit (7,0 mg/ml) und Natriumsulfit (3,0 mg)), steigern läßt und die Trübung in der Flüssigkeitsprobe schneller eintritt. Alternativ kann die Reaktionsgeschwindigkeit und damit die Trübung durch steigende Cystein-Konzentrationen erhöht werden. Vorteilhafterweise kann somit die Geschwindigkeit und Intensität der gewünschten Trübung durch Variierung der betreffenden organischen und anorganischen Reagenzien leicht gesteuert werden.

Die für das erfindungsgemäße Verfahren zu verwendende wäßrige Lösung, in der die enzymatisch und/oder chemische Behandlung einer Probe Keratin enthaltenden Hautanhangsgebilde stattfindet, kann in Abhängigkeit von den gewählten Reaktionspartnern (Enzyme und/oder organische und/oder anorganische Reagenzien) für die enzymatische und/oder chemische Behandlung einen pH Wert im Bereich zwischen 5,0 bis 11,0 aufweisen, vorzugsweise einen pH Wert im Bereich zwischen 5,0 und 9,0, insbesondere zwischen 5,5 und 7,5. Vorteilhafte pH-Bereiche zwischen 5,5 und 7,5 können beispielsweise bei Verwendung der Proteinase K, pH-Bereiche zwischen 6,0 und 7,5 bei Verwendung der Pronase E und pH-Bereiche zwischen 6,0 und 7,5 im Falle von Papain angegeben werden. Allgemein kann als pH-Wert ein Bereich zwischen 5,5 und 7,5 gelten, in dem vorteilhafte proteolytische oder hydrolytische Zersetzungen von Proben Keratin enthaltender Hautanhangsgebilde und damit geeignete Trübungen erzielt werden können. Für die genannten Enzym-Beispiele kann ein pH-Wert von 6,5 als Richtwert zugrunde gelegt werden. Wenn andere Enzyme zur Anwendung kommen sollen, müssen selbstverständlich die pH-Optima entsprechend gewählt werden.

Vorteilhafterweise kann der Reaktionsansatz bzw. die Flüssigkeitsprobe, in dem die enzymatische und/oder hydrolytische Behandlung einer Probe Keratin enthaltender Hautanhangsgebilde stattfindet und als wäßrige Lösung vorliegt, ein Puffersystem enthalten, beispielsweise einen Phosphatpuffer. Zum Beispiel eignet sich für den genannten Zweck ein Natriumphosphatpuffer (50 mM) mit einem pH-Wert von 6,5.

Der Temperaturbereich, in dem die enzymatische und/oder chemische Reaktion der Probe Keratin enthaltender Hautanhangsgebilde in der wäßrigen Lösung erfolgt, orientiert sich ebenfalls an den ausgewählten Reaktionspartnem für die enzymatische und/oder hydrolytische Behandlung der Probe Keratin enthaltender Hautanhangsgebilde. Der zu wählende Temperaturbereich kann zwischen 20°C und 100°C liegen, vorteilhafterweise zwischen 30°C und 60°C, insbesondere zwischen 40°C und 50°C. Es konnte festgestellt werden, daß die Geschwindigkeit und Stärke der Zersetzung von Keratin enthaltenden Hautanhangsgebilden, insbesondere von Haaren, mit steigender Temperatur steigt und die Trübung entsprechend zunahm. Bei einer Temperatur von 50°C und beispielsweise einem Reaktionsgemisch bestehend aus einem proteolytischen Enzym (zum Beispiel Papain) und einem Reduktionsmittel (zum Beispiel Cystein) in einem Phosphatpuffer (50 mM) wurde eine Haarprobe schnell zersetzt. Auch hier, wie bei der Wahl der Reaktionspartner (beispielsweise Variierung der Mengen an Cystein und Sutfiten), kann mit der Wahl der Reaktionstemperatur die Veränderung der Trübung in vorteilhafter Weise gesteuert werden.

Die gewählte Menge und Länge an zu untersuchenden Proben Keratin enthaltender Hautanhangsgebilde (Haar-, Woll-, Faser-, Federproben) ist vorteilhafterweise so zu wählen, daß sie sich an einem Vergleichswert (zum Beispiel gesundes Material) mit definierter Menge (in mg) und/oder Länge (in cm) orientiert. In Abhängigkeit des Volumens des Reaktionsgefäßes, in dem die der Erfindung zugrundeliegende enzymatische und/oder chemische Behandlung stattfindet, können Menge und Länge der zu verwendenden Proben weit variieren. Zweckmäßigerweise kann die Menge der Probe Keratin enthaltender Hautanhangsgebilde zwischen 0,01 mg und 100 mg betragen, insbesondere zwischen 0,5 mg und 50 mg, vorteilhafterweise zwischen 1,0 mg und 10 mg. Im Falle von Haaren (oder Wolle oder Fasern) kann die Länge der Haarprobe zwischen 0,05 cm und 5,0 cm, insbesondere zwischen 0,1 cm und 1,0 cm, vorteilhafterweise zwischen 0,1 cm und 0,5 cm, gewählt werden. Geringere Mengen (0,1 bis 10 mg) und Längen (0,1 bis 0,5 cm) sind vorteilhafterweise dann zu wählen, wenn die Messung der Trübung in kleinen Volumina (beispielsweise in kleinen Reagenzröhrchen oder Küvetten für photometrische Messungen) erfolgen soll. Die beispielhaften genannten Mengen korrespondieren mit den oben beispielhaft genannten Mengen an eingesetzten Reaktionspartner für die enzymatische und/oder chemische Behandlung. Selbstverständlich können die Mengen und Längen der Proben Keratin enthaltender Hautanhangsgebilde vergrößert oder verringert werden, je nach den vorliegenden Bedingungen und vorzunehmenden Verfahrensweisen. Grundsätzlich ist es von Vorteil, die Haarlänge nicht über einen Bereich von 1,0 cm zu wählen, da allgemein eine geringere Haarlänge den proteolytischen und/oder hydrolytischen Abbau der Haare beschleunigen kann und somit innerhalb kürzester Zeit (< 10 Minuten) erfindungsgemäß verwertbare Trübungen erzielt werden können.

Die Zeitspanne, innerhalb derer eine geeignete Trübung für die Bestimmung von Zuständen von Keratin enthaltenden Hautanhangsgebilden eintritt, variiert in Abhängigkeit der Wahl der bereits genannten Parameter (Reaktionspartner, pH-Wert, Temperatur, Menge und Länge der Probe Keratin enthaltender Hautanhangsgebilde. Die Dauer der enzymatischen und/oder chemischen Behandlung der Probe Keratin enthaltender Hautanhangsgebilde kann zwischen 1 Minute und 30 Minuten betragen, vorzugsweise zwischen 5 Minuten und 20 Minuten. Verschiedene Versuche haben gezeigt, daß bereits innerhalb von 10 Minuten eine für die Auswertung und Bestimmung beispielsweise des Haarzustandes ausreichende Trübung mit den erfindungsgemäßen Verfahren erzielt werden kann.

Da die Versuche zur Durchführung der vorliegenden Erfindung insbesondere an Haaren vorgenommen wurde, soll im weiteren am Beispiel Haar die Erfindung näher erläutert werden, ohne den Gegenstand der Erfindung damit einzuschränken. Die im folgenden beschriebenen Maßnahmen und angegebenen Wert- und Mengenangaben sind ohne weiteres auch auf andere Keratin enthaltende Hautanhangsgebilde anzuwenden und zu übertragen.

Es konnte allgemein festgestellt werden, daß in Gegenwart eines Enzyms durch geeignete Wahl an betreffenden organischen oder anorganischen chemischen Reagenzien, pH-Wert und Temperatur das Ausmaß der proteolytischen Zersetzung von Haarproben in einem weiten Bereich variiert. Eine höhere Reaktionsgeschwindigkeit äußerte sich hierbei in einer Verminderung der maximal erreichbaren Trübung. Bei einer langsameren Reaktion innerhalb von 10 bis 15 Minuten nach Zugabe des Enzyms (beispielsweise bei Verwendung von Papain und 20 mg/ml Cystein, pH 5,5, 50°C oder Papain und 10 mg/ml DTE, pH 6,0, 45°C) wurde ein zunehmend linearer Kurvenverlauf erhalten, was eine Auswertung erleichtern kann. Demzufolge ist es besonders vorteilhaft, wenn die Dauer der Reaktion bis zur maximal erreichbaren Trübung zwischen 5 bis 20 Minuten, insbesondere zwischen 7 bis 15 Minuten beträgt. Es hat sich herausgestellt, daß eine derartige Reaktionsführung vorteilhaft ist, wenn die zu untersuchenden Haarproben stärkere Haarschädigungen erwarten lassen. Beispielhaft können hierfür ganz besonders bevorzugte Bedingungen für die enzymatisch/chemische Behandlungen von Haarproben genannt werden: 5,0 mg Haare einer Länge von ca. 0,1 cm, Papain (21,2 U) plus Cystein (20 mg/ml), pH 6,5, 50°C oder Papain (21,2 U) plus DTE (10 mg/ml), pH 6,2, 50°C.

Für die Untersuchung und Auswertung der erfindungsgemäß erhaltenen Trübung in der Flüssigkeitprobe kann es sich als nachteilig erweisen, wenn in der Flüssigkeitsprobe nach erfolgter enzymatischer und/oder chemischer Behandlung der Haare nicht zersetzte Haarbestandteile vorhanden sind. Das kann sich beispielsweise bei Anwendung physikalischer Meßmethoden, insbesondere bei turbidimetrischen Messungen mittels eines Photometers, störend auswirken und zu verfälschten Resultaten führen. Es kann daher von Vorteil sein, nicht durch die enzymatische und/oder chemische Behandlung zersetzte Haarbestandteile vor der Untersuchung der Flüssigkeitsprobe zu entfernen oder während der Untersuchung entfernt zu halten und die Untersuchung und Auswertung der Flüssigkeitsprobe in Abwesenheit nicht zersetzter Haarbestandteile durchzuführen. In vielen Fällen wird es genügen, daß insbesondere bei Anwendung von meßtechnischen Verfahren (beispielsweise Extinktions- oder Trübungsmessungen mittels eines Photometers) vornehmlich derjenige Bereich der Flüssigkeitsprobe von nicht zersetzten Haarbestandteilen befreit wird, durch den ein Strahlengang zur Messung der Trübung erfolgt. Bodensätze an nicht zersetzten Haarbestandteilen sind in diesen Fällen ohne erhebliche Bedeutung.
Die Entfernung nicht zersetzter Haarbestandteile vor der Untersuchung der Flüssigkeitsprobe kann durch physikalische Trennverfahren erfolgen, beispielsweise durch Filtration, Zentrifugation, Sedimentation oder durch Verwendung von geeigneten mechanischen Trennmitteln wie beispielsweise, Siebe, Netze, Gitter oder Filter. Solche Verfahren lassen sich leicht mit Reagenzröhrchen, Meßzellen oder Meßküvetten anwenden oder entsprechende mechanische Trennmittel können in solche Röhrchen, Zellen oder Küvetten dauerhaft oder herausnehmbar eingebaut werden. Beispielsweise kann mit in die Meßzelle eingebauten mechanischen Trennmitteln erreicht werden, daß der Bereich der Flüssigkeitsprobe, in dem die enzymatische und/oder hydrolytische Behandlung der Haare stattfindet, von jenem Bereich der Flüssigkeitsprobe getrennt ist, der für die Trübungsmessung verwendet wird. Hierbei kann es zweckmäßig sein, daß eine Meßzelle mit integrierter oder hineinragender Vermischvorrichtung eine gieichmäßige Verteilung der die Trübung verursachenden Haarbestandteile bewirkt.

Demzufolge besteht erfindungsgemäß ein bevorzugtes Verfahren darin, daß nicht durch die enzymatische und/oder chemische Behandlung zersetzte Haarbestandteile (bzw. Bestandteile Keratin enthaltender Hautanhangsgebilde) vor der Untersuchung der Flüssigkeitsprobe entfernt werden oder während der Untersuchung entfernt gehalten werden, und daß die Untersuchung und Auswertung der Flüssigkeitsprobe in Abwesenheit nicht zersetzter Haarbestandteile (bzw. Keratin enthaltender Hautanhangsgebilde) durchgeführt wird.
Vorteilhafterweise kann der für die Messung und Untersuchung der Flüssigkeitsprobe verwendete lichtdurchlässige Behälter (Reagenzröhrchen, Meßzelle, Meßküvtte), der aus Glas oder Kunststoff bestehen kann, zusätzlich mit einer Vorrichtung zur Thermostatisierung der Flüssigkeitsprobe ausgestattet sein. Eine solche Vorrichtung kann beispielsweise aus einer Heizfolie oder einem beheizten Wasserbad bestehen.

Die nach der enzymatischen und/oder chemischen Behandlung der Keratin enthaltenden Hautanhangsgebilde erhaltene Flüssigkeitsprobe kann optisch oder visuell untersucht werden. Die Untersuchung kann beispielsweise mit bloßem Auge durchgeführt werden oder mittels dafür geeigneter physikalischer Meßmethoden mit Hilfe hierfür geeigneter Vorrichtungen und Mittel.

Die Untersuchung mit bloßem Auge kann dadurch erfolgen, daß eine Flüssigkeitsprobe beispielsweise aus einer entsprechend behandelten Haarprobe mit einer aus ungeschädigten Haar erhaltenen Flüssigkeitsprobe (Vergleichs- oder Nullwert) vorzugsweise im Durchlicht verglichen wird. Ein solches Verfahren kommt insbesondere dann in Frage, wenn beispielsweise nur eine erste grobe Einschätzung einer Haarschädigung gewünscht wird.

Die Untersuchung mittels physikalischer Meßmethoden wird erfindungsgemäß bevorzugt, da diese eine genauere Auswertung und Bestimmung einer Schädigung von Keratin enthaltenden Hautanhangsgebilden (zum Beispiel Haarschädigungen) erlaubt. insbesondere wird ein Verfahren bevorzugt, wonach die Untersuchung und Auswertung der Flüssigkeitsprobe über Trübungsmessung erfolgt. Hierfür können alle die dem Fachmann bekannten optischen Verfahren angewendet werden, soweit sie Messungen von Flüssigkeiten mit ungelösten, suspendierten Teilchen gestatten. Hierzu zählen beispielsweise Verfahren auf Basis von Lichtstreuungsmessungen, nephelometrischen Messungen (Tyndallometrie), Fluoreszenzstrahlungsmessungen, Durchlichtmessungen bzw. Extinktionsmessungen, turbidimetrischen Messungen (Turbidimetrie). Die Trübungsmessung (turbidimetrische Messung) der erhaltenen Flüssigkeitsprobe wird erfindungsgemäß bevorzugt.
Für die Durchführung des erfindungsgemäßen Verfahrens auf Grundlage physikalischer Meßmethoden können alle die dem Fachmann bekannten und kommerziell erhältlichen Vorrichtungen und Mittel verwendet werden. Hierunter kommen insbesondere Photometer in Betracht. Bevorzugt wird die Trübungsmessung mittels eines Photometers bzw. Trübungsphotometers.
Ein bevorzugtes Verfahren besteht demgemäß darin, daß die betreffende Flüssigkeitsprobe über Trübungsmessung mittels eines Photometers untersucht und ausgewertet wird. Anschließend kann der Zustand der Keratin enthaltenden Hautanhangsgebilde (Haare bzw. die Schädigung der Haare) gegenüber einem Vergleichswert oder Nullwert (zweckmäßigerweise aus nicht geschädigtem Haar) leicht bestimmt werden.

Erfindungsgemäß umfaßt wird aber auch die Verwendung eines Trübungsphotometers zur Festellung und Bestimmung des Zustandes von Keratin enthaltenden Hautanhangsgebilde in einem der dieser Erfindung zugrundeliegenden Verfahren.

Im Falle der Untersuchung, Auswertung und Bestimmung des Zustandes Keratin enthaltender Hautanhangsgebilden mittels Trübungsmessung sind die allgemein gültigen Trübungseinheiten zugrunde zu legen. Als Trübungseinheiten können entweder die durch Messung der Schwächung der durch die Flüssigkeitsprobe durchgehenden Strahlung (FAU, "Formazine Attenuation Unit") ermittelten Größen zugrunde gelegt werden, oder die durch Messung der Intensität der gestreuten Strahlung (FNU, "Formazine Nephelometric Unit"). Der über FNU ermittelten Größen entspricht die deutsche "Trübungseinheit Formazin (TE/F).

Die Untersuchung und Auswertung der Flüssigkeitsprobe kann gemäß dem erfindungsgemäßen Verfahren während und/oder nach der enzymatischer und/oder chemischer Behandlung der Probe Keratin enthaltender Hautanhangsgebilde erfolgen. Die Untersuchung und Auswertung der Flüssigkeitsprobe während und/oder nach der enzymatischen und/oder chemischen Behandlung der Probe kann entweder indirekt und diskontinuierlich oder direkt und kontinuierlich (on-line), erfolgen. Beim diskontinuierlichen Verfahren kann zu bestimmten Zeitabständen die Trübung der Flüssigkeitsprobe durch Einzetwerte bestimmt oder gemessen werden.

Das kontinuierliche Verfahren, bei der die Trübung oder die Veränderung der Trübung in der Flüssigkeitsprobe direkt und fortlaufend (on-line) verfolgt werden kann, hat die Vorteile eines geringeren Zeitaufwandes durch Einsparen von beispielsweise Pipettierarbeiten. Außerdem besteht die Möglichkeit einer kinetischen Auswertung der erhaltenen Daten. Hierdurch kann das erfindungsgemäße Verfahren weiter verkürzt werden, so daß eine Auswertung und Bestimmung des Zustandes der Keratin enthaltenden Hautanhangsgebilde in weniger als 10 Minuten ermöglicht wird.
Das kontinuierliche Verfahren wird erfindungsgemäß bevorzugt. Ein solches Verfahren läßt sich mittels einer entsprechend geeigneten Vorrichtung durchführen, die aus einem Photometer besteht in Kombination mit einer Heizung, welche das für die Trübungsmessung erforderliche, lichtdurchlässige Reaktionsgefäß (Meßzelle, Röhrchen, Küvette,) beheizt, einer in das Reaktionsgefäß ragenden oder darin integrierten Vermischvorrichtung und einem steuerbaren Antrieb für die Bewegung der Vermischvorrichtung, welcher vorzugsweise ein Elektromotor sein kann.

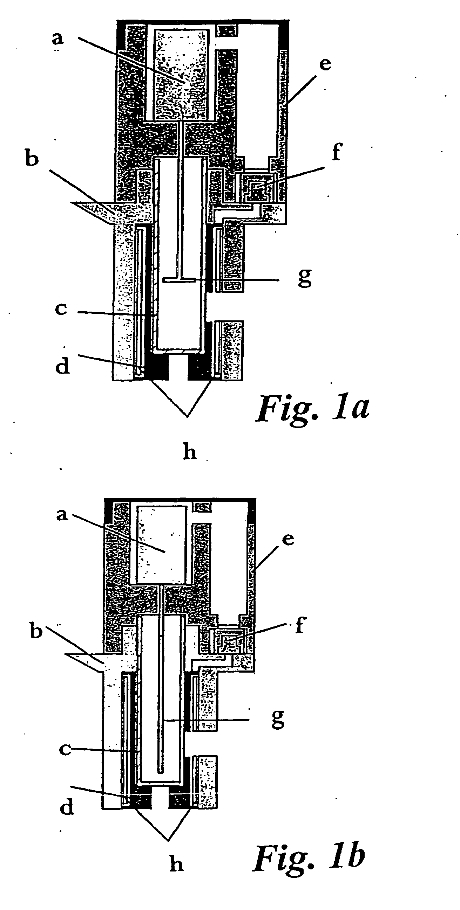

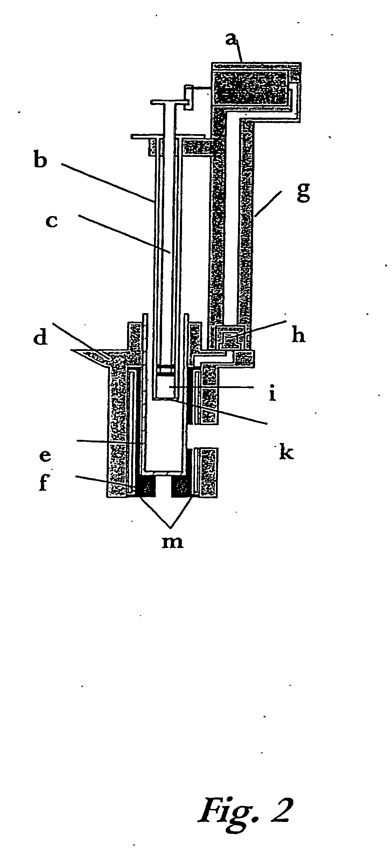

Die Heizung erfolgt vorzugsweise über eine geregelte Thermostatisierung (Thermoelement) und kann beispielsweise aus einer Heizfolie bestehen, welche in Kontakt mit dem Reaktionsgefäß steht.
Die Vermischvorrichtung, welche vorzugsweise durch einen mit dem Photometer räumlich verbundenen Elektromotor regelbar angetrieben werden kann, kann aus einem Rührer bestehen oder aus einer Spritze oder Einmalspritze, die in die Meßzelle bzw. in die darin enthaltene Reaktionsflüssigkeit ragen. Der Rührer kann als Rotor mit entsprechenden Rührblättern ausgestaltet sein oder ein eckiger oder runder Stab aus Metall, Glas oder Kunststoff darstellen, welcher Einkerbungen aufweisen kann, beispielsweise ein Bohrer. Die Rührvorrichtung kann aber auch aus einem Magnetrührer bestehen, wobei die Rührbewegung durch einen in der Reaktionsflüssigkeit befindlichen Rührkörper hervorgerufen wird. Im Falle der Spritze kann diese vorteilhafterweise am unteren Ende mit einem Gitter, Sieb oder Netz ausgestattet sein. Der Stempel oder Kolben der Spritze kann durch eine Auf- und Abbewegung die Haarprobe bzw. die Flüssigkeitsprobe in ständiger Bewegung halten. Damit wird gewährleistet, daß die Probe Keratin enthaltender Hautanhangsgebilde in der Reaktionsflüssigkeit ständig bewegt wird und die enzymatisch und/oder chemische Behandlung der Probe Keratin enthaltender Hautanhangsgebilde unter optimalen Bedingungen ablaufen kann. Das Volumen der Spritze ist in diesem Falle der Reaktionsraum, in dem die enzymatische und/oder chemische Behandlung der Probe Keratin enthaltender Hautanhangsgebilde erfolgt. Die Auf- und Abbewegung des Stempels kann durch einen geregelten Elektromotor erfolgen. In Fig.1a und b und Fig. 2 sind beispielhaft geeignete Vorrichtungen schematisch dargestellt.
Es ist dem Fachmann bekannt, daß proteolytische und hydrolytische Reaktionen durch die Intensität der Vermischung der Reaktionsflüssigkeit beeinflußt werden können und von der Geschwindigkeit des Rührers bzw. dem Bewegungsgrad der Vermischvorrichtung abhängen können. Allgemein kann davon ausgegangen werden, daß die Geschwindigkeit und Intensität der vorliegenden Erfindung zugrundeliegende proteolytische oder hydrolytische Zersetzung der Probe an Keratin enthaltenden Hautanhangsgebilden mit der Umdrehungs- bzw. Bewegungsgeschwindigkeit der Vermischvorrichtung zunimmt. Je nachdem innerhalb welcher Zeit bestimmte Trübungen erzielt werden sollen, kann dieser Parameter variiert werden. Umdrehungen mit einem Magnetrührer oder mit einem stabförmigen Rührer mit Rührblättern oder Einkerbungen zwischen 100 und 10000 Umdrehungen pro Minute (UpM), insbesondere zwischen 500 und 5000 UpM, vorzugsweise zwischen 500 und 2000 UpM, haben sich als zweckmäßig herausgestellt. Auf- und Abbewegungen eines Stempels oder Kolbens in einer spritzenförmigen Vermischvorrichtung können zwischen 10 und 500, insbesondere zwischen 50 und 200, betragen.

Demzufolge besteht ein bevorzugtes Verfahren darin, daß die Probe Keratin enthaltender Hautanhangsgebilde während der enzymatischen und/oder chemischen Behandlung einer zusätzlichen mechanischen Belastung unterworfen wird.

Wenn das erfindungsgemäße Verfahren und die damit verbundene Feststellung, Auswertung und Bestimmung des Zustandes Keratin enthaltender Hautanhangsgebilde weitgehend automatisiert werden soll, kann die Untersuchung und Auswertung einer Flüssigkeitsprobe unter Zuhilfenahme elektronischer Mittel durchgeführt werden. Demgemäß besteht das erfindungsgemäße Verfahren auch darin, daß die Untersuchung und/oder Auswertung der Flüssigkeitsprobe computergesteuert ist und mit Hilfe eines Computers erfolgt.
Für diese Zwecke steht die beschriebene Photometer-Vorrichtung vorteilhaft in Verbindung mit einem Rechner, internem Prozessor oder "Personal Computer". Damit kann es ermöglicht werden, die durch die Trübungsmessung erhaltenen Daten zu speichern und jederzeit auszuwerten.

Zusätzlich kann in vorteilhafter Weise ein Schreiber oder Drucker an ein Photometer angeschlossen werden, um eine graphische Darstellung der Trübungsverläufe zu erhalten oder Zahlenwerte auszudrucken. Ebenso ist es möglich, einen Bildschirm oder ein "Display" mit dem Photometer zu verbinden, um Graphiken oder Zahlenwerte zu erhalten, die direkt abgelesen werden können. Derartige Daten lassen sich mittels eines ebenfalls angeschlossenen Rechners (Computer) speichern und beispielsweise für vergleichende Zwecke jederzeit abrufen und wahlweise über einen daran angeschlossenen Drucker ausdrucken. Hierzu kann jeder übliche Computer, insbesondere ein üblicher Personal Computer (PC), verwendet werden.

Ein weiterer Gegenstand des erfindungsgemäßen Verfahrens ist daher dadurch gekennzeichnet, daß die Untersuchung und Auswertung der Flüssigkeitsprobe unter Zuhilfenahme eines Computers und/oder eines Schreibers und/oder eines Bildschirms (Display) und/oder eines Druckers erfolgt.

Die Bereitstellung einer Vorrichtung zur Feststellung, Bestimmung und Auswertung eines Zustandes Keratin enthaltender Hautanhangsgebilde, welche in Kombination die Bestandteile Photometer mit Reaktionsgefäß (beispielsweise eine Meßzelle, eine Küvette oder ein Reagenzröhrchen), einer Heizung zur Erwärmung des Reaktionsgefäß des Photometers, einer in das Reaktionsgefäß ragenden oder darin integrierten Vermischvorrichtung und einem steuerbaren Antrieb für die Bewegung der Vermischvorrichtung aufweist, ist daher für die Durchführung des erfindungsgemäßen Verfahrens von Vorteil.

Demzufolge ist Gegenstand der Erfindung auch die Verwendung einer Vorrichtung zur Feststellung, Bestimmung und Auswertung eines Zustandes Keratin enthaltender Hautanhangsgebilde bestehend aus einem Photometer mit Reaktionsgefäß und Adapter oder Halterung dafür in Kombination mit einer Heizung zur Erwärmung des Reaktionsgefäßes, einer Vermischvorrichtung für die Vermischung der Flüssigkeitsprobe im Reaktionsgefäß und einem steuerbaren Antrieb für die Vermischvorrichtung. Zur Konstanthaltung der Temperatur im Reaktionsgefäß kann die Heizung mit einem Thermoelement verbunden sein, wobei die Heizung als Heizfolie ausgestaltet sein kann. Eine solche Heizfolie kann vorzugsweise in die bei Photometem üblicherweise vorhandenen Adaptern oder Halterungen für die erfindungsgemäß zu verwendenden Reaktionsgefäße, insbesondere Küvetten oder Reagenzröhrchen, integriert sein. Die Vermischvorrichtung kann aus einer Spritze mit Stempel oder Kolben oder einem Rührer bestehen, welche über einen mechanischen, elektrischen oder magnetischen Antrieb angetrieben und bewegt werden können oder aus einem Ultraschall erzeugenden Gerät, so daß die Vermischung mittels Ultraschall erfolgen kann.

Eine solche Vorrichtung kann vorteilhafterweise mit einem Schreiber, Drucker, Bildschirm (Display) oder Rechner (Computer), internen Prozessor oder Personal Computer (PC) funktionell verbunden sein.

Für die praktische Handhabung kann es von Vorteil sein, wenn die für die erfindungsgemäße Verwendung geeignete Vorrichtung als mobile und kompakte Baueinheit vorliegt. Insbesondere kann der Rechner vorteilhafterweise in Form eines Handgerätes ausgestaltet sein, welches räumlich getrennt von der stationären Photometereinheit, vorzugsweise in Verbindung mit einem Schreiber, Drucker oder einer Bildschirmanzeige, mobil benutzt werden kann.

Da die Verwendung einer Vorrichtung, wie beschrieben, für den erfindungsgemäßen Zweck von Vorteil ist, umfaßt die vorliegende Erfindung auch die Verwendung der beschriebenen Vorrichtung zur Feststellung und Bestimmung des Zustandes von Keratin enthaltenden Hautanhangsgebilden in einem der erfindungsgemäßen Verfahren.

Für die Durchführung des erfindungsgemäßen Verfahrens ist es zweckmäßig, wenn sowohl die hierfür geeignete Vorrichtung als auch die dazu notwendigen Reagenzien in Kombination als eine Einheit oder als ein Kit vorliegen. Ein solcher Kit kann vorteilhafterweise bestehen aus einer Vorrichtung wie beschrieben zusammen mit einer Pufferlösung, mindestens einem Enzym und mindestens einem proteolytisch oder hydrolytisch wirkenden und keratinolytisch wirksamen und/oder reduzierenden chemischen Reagenz. Eine solche Kombination (Kit) hat den Vorteil, daß alle Einzelkomponenten aufeinander abgestimmt sind, das Verfahren für den Anwender routinemäßig und leicht durchzuführen ist und immer reproduzierbare und vergleichbare Ergebnisse erzielt werden können.

Ein weiterer Gegenstand der vorliegenden Erfindung liegt daher in der Verwendung eines Kits zur Untersuchung, Auswertung und Bestimmung des Zustandes von Keratin enthaltenden Hautanhangsgebilden, umfassend eine Pufferlösung, mindestens ein Enzym und/oder mindestens ein proteolytisch oder hydrolytisch wirkendes und keratinolytisch wirksames und/oder reduzierendes chemisches Reagenz. Zweckmäßigerweise kann dieser Kit zusätzlich eine Probe an Keratin enthaltenden Hautanhangsgebilden, beispielsweise eine Haarprobe aus kosmetisch nicht behandeltern Haar, und/oder einer Flüssigkeitsprobe als Standard-, Blind- oder Nullwert enthalten. Ein solcher Kit kann aber auch zusätzlich zu den genannten Bestandteilen eine der vorliegenden Erfindung zugrundeliegende Vorrichtung umfassen.
Die Verwendung eines der beschriebenen Kits zur Festellung und Bestimmung des Zustandes von Keratin enthaltenden Hautanhangsgebilden in einem der erfindungsgemäßen Verfahren ist vorteilhaft und daher Gegenstand der vorliegenden Erfindung.

### Legenden zu den Abbildungen:

- Fig.1 a und 1 b:: Zwei Vorrichtungen zur direkten (on-line) Bestimmung der Trübung von Haarproben in Kombination mit einem Photometer, worin bedeuten: a = Elektromotor, b = Adapter für Glasküvette, c = Glasküvette, d = Aluminiumblock, e = Steckaufsatz, f = Steckkontakt, g = Rührer, h = Heizfolie.
- Fig. 2:: Alternative Vorrichtung zur direkten (on-line) Bestimmung der Trübung von Haarproben in Kombination mit einem Photometer, worin bedeuten: a = Elektromotor, b = Einmalspritze, c = Stempel, d = Adapter für Glasküvette, e = Glasküvette, f = Aluminiumblock, g = Steckaufsatz, h = Steckkontakt, i = Reaktionsraum, k = Gitter, m = Heizfolie.

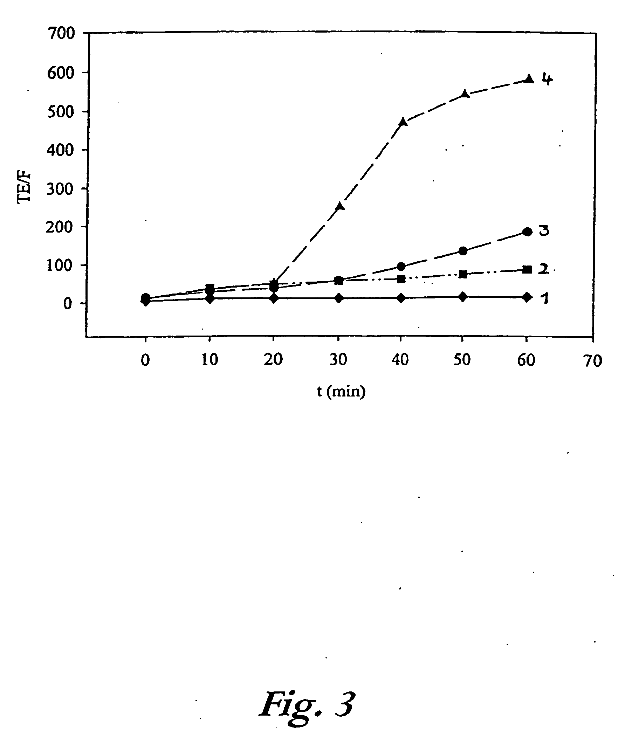

- Fig. 3:: Trübungsphotometrische Bestimmung der proteolytischen Zersetzung von Haarproben zweier verschiedener Oxidationsstufen. 1 = Blindwert, nicht oxidiertes Haar, 2 = nicht oxidiertes Haar, 3 = 1-fach oxidiertes Haar, 4 = 3-fach oxidiertes Haar. Versuchsbedingungen: 50 mg Haare (1,0 cm lang); 100 µl Papain (21,2 U); 5,0 ml 50 mM Natriumphosphatpuffer, pH 6,5; (Reaktionsvolumen, Flotte); 0,74 mg/ml L-Cystein; 7,0 mg/ml Na₂S₂O₅; 3,0 mg/ml Na₂SO₃; 45°C; Rührgeschwindigkeit 600 UpM (Magnetrührer). Es wurde jeweils eine 1-fach Bestimmung aufgetragen.

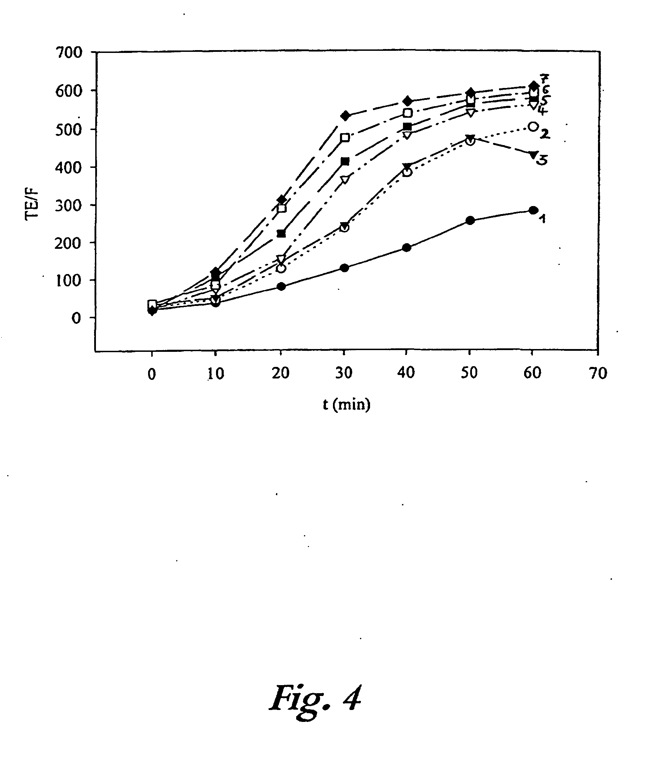

- Fig. 4:: Trübungsphotometrische Bestimmung der proteolytischen Zersetzung von Haarproben von sieben verschiedenen Oxidationsstufen. 1 = 1-fach, 2 = 2-fach, 3 = 3-fach, 4 = 4-fach, 5 = 5-fach, 6 = 6-fach, 7 = 7-fach oxidiertes Haar. Versuchsbedingungen wie bei Fig. 3 mit der Ausnahme, daß die Haare eine Länge von 0,5 cm aufwiesen. Es wurde jeweils eine 1-fach Bestimmung aufgetragen.

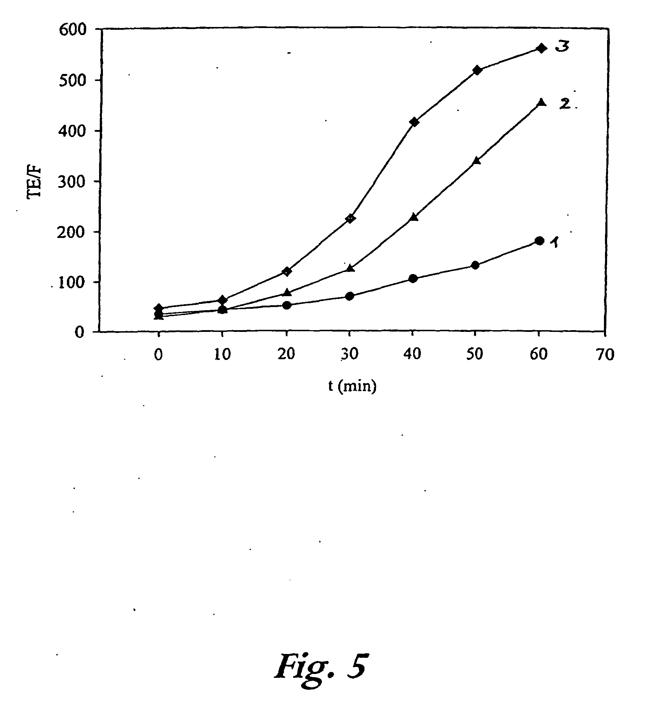

- Fig. 5:: Trübungsphotometrische Bestimmung der proteolytischen Zersetzung von verschieden oft dauergewellten Haarproben. 1 = 1-fach, 2 = 3-fach, 3 = 6-fach dauergewelltes Haar. Versuchsbedingungen wie bei Fig. 3 mit der Ausnahme, daß die Haare eine Länge von 0,5 cm aufwiesen. Es wurde jeweils eine 1-fach Bestimmung aufgetragen.

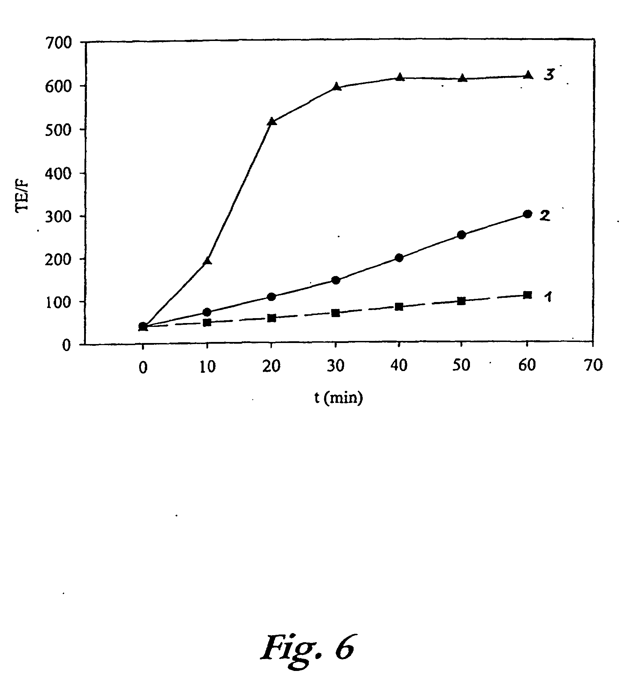

- Fig. 6:: Optimierung der proteolytischen Zersetzung von Haarproben verschiedener Oxidationsstufen. 1 = nicht oxidiertes Haar, 2 = 1-fach, 3 = 3-fach oxidiertes Haar. Versuchsbedingungen wie bei Fig. 3, mit den Ausnahmen, daß 50 mg Haare der Länge von 0,3 cm lang und 500 µl Papain (108 U) verwendet wurden. Es wurde jeweils eine 1-fach Bestimmung aufgetragen.

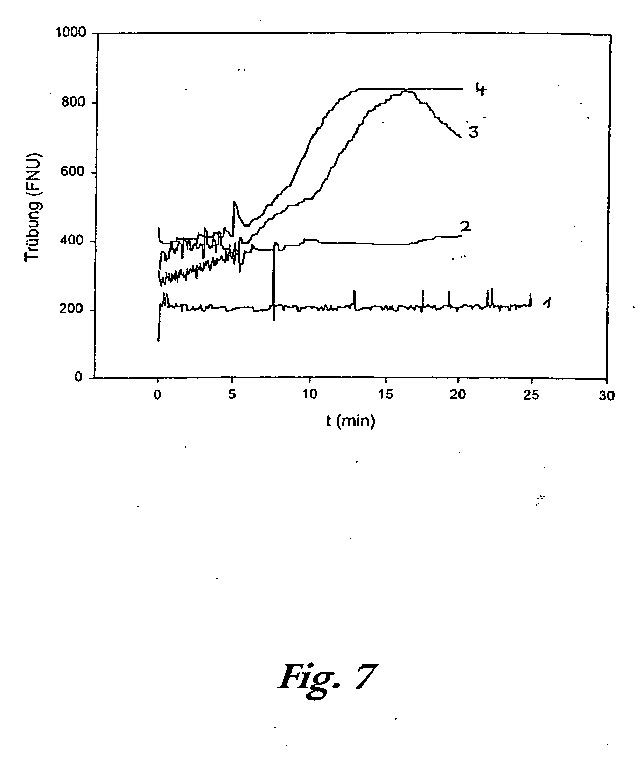

- Fig. 7:: Direkte, kontinuierliche (on-line) Messung der Trübung bei Proteolyse von oxidierten Haarproben. 1 = nicht oxidiertes Haar, 2 = 1-fach, 3 = 2-fach, 4 = 3-fach oxidiertes Haar. Versuchsbedingungen: 5,0 mg Haarprobe (0,1 cm lang); 100 µl (21,2 U) Papain; 3,0 ml 50 mM Natriumphosphatpuffer, pH 6,2 (Reaktionsvolumen, Flotte); 10 mg/ml DTE; 50°C; Umdrehungen des Rührers 1000 UpM.

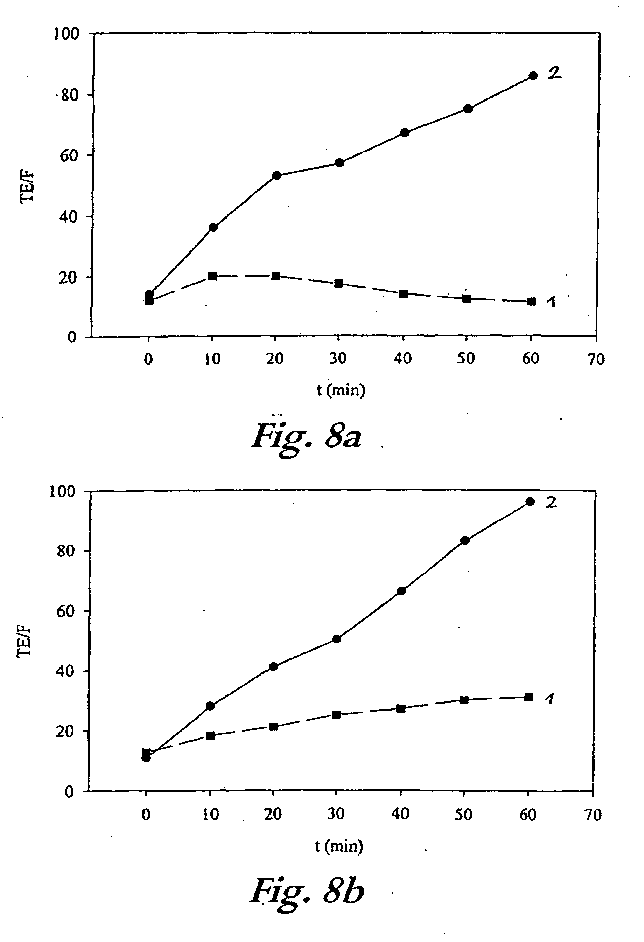

- Fig. 8:: Vergleich der proteolytischen Zersetzung von bewetterten und unbewetterten, nicht behandelten Haarproben von dunkelbraunen und blonden Haaren. 8a: dunkelbrauner Haarzopf, 1 = Haaransatz, 2 = Haarspitze; 8b: blonder Haarzopf, 1 = Haaransatz, 2 = Haarspitze. Versuchsbedingungen für 8a und 8b: 50,0 mg Haarprobe (0,5 cm lang); 100 µl (21,2 U) Papain; 5,0 ml 50 mM Natriumphosphatpuffer, pH 6,5 (Reaktionsvolumen, Flotte); 0,74 mg/ml L-Cystein; 7,0 mg/ml Na₂S₂O₅; 3,0 mg/ml Na₂SO₃; 45°C; Rührgeschwindigkeit 600 UpM (Magnetrührer). Es wurde jeweils eine 1-fach Bestimmung aufgetragen.

Die folgenden Beispiele sollen die Erfindung näher veranschaulichen.

### Beispiel 1: Oxidative Behandlung der Haarproben

Zur Herstellung verschieden stark oxidativ geschädigter Haare wurden Haare von mitteleuropäischen Frauen nach Farbe und Dichte sortiert und ohne Ansatz und Spitze zu Kämmsträhnen (jeweils 3 g Haare, 15,0 cm lang) zusammengefaßt. Tabelle 1 zeigt die Oxidationsstufen und die jeweilige chemische Behandlung der Haare.

**Tabelle 1**

| **Oxidationsstufe** | **chemische Behandlung** |
|---|---|
| 1-fach oxidiert | 30 Minuten mit 6 % H₂O₂ |
| 2-fach oxidiert | 2 x 30 Minuten mit 6 % H₂O₂ |
| 3-fach oxidiert | 3 x 30 Minuten mit 6 % H₂O₂ |
| 4-fach oxidiert | 4 x 30 Minuten mit 6 % H₂O₂ |
| 5-fach oxidiert | 5 x 30 Minuten mit 6 % H₂O₂ |
| 6-fach oxidiert | 6 x 30 Minuten mit 6 % H₂O₂ |
| 7-fach oxidiert | 7 x 30 Minuten mit 6 % H₂O₂ |

Das Wasserstoffperoxid wurde mit einem Bleichpulver (50,0 % Ammoniumpersulfat, 23,0 % NaHCO₃, 25,5 % Natriumsilikat, 0,5 % SiO2 (Aerosil 300, Degussa), 1,0 % EDTA) 3 : 1 vermischt.

### Beispiel 2: Dauerwellbehandlung der Haarproben

Zur Herstellung verschieden stark durch Dauerwellbehandlung geschädigter Haare wurden Kämmsträhnen gleicher Herkunft und Art wie in Beispiel 1 beschrieben einfacher, dreifacher und sechsfacher Dauerwellbehandlungen unterworfen.
Hierzu wurden die Haarsträhnen gut durchgefeuchtet, mit einer Saugserviette abgetupft und über Nacht bei einer relativen Luftfeuchtigkeit von 95 - 100 % konditioniert. Auf die konditionierten Haare wurde Dauerwellflüssigkeit mit einem Gehalt an Thioglykolsäure von 8% (w/v) im Verhältnis 1 : 1,2 zum Trockengewicht der Haarsträhne gleichmäßig aufgetragen. Die Strähnen wurden in eine Folientasche gelegt und bei 45°C im Trockenschrank für 15 Minuten gehalten. Nach Beendigung der Einwirkzeit wurden die Strähnen 5 Minuten unter fließendem 37°C warmen Wasser gewaschen und danach für 3 Minuten in eine Fixierlösung (3% H₂O₂) getaucht. Anschließend wurden die Strähnen aus dem Fixierbad genommen und für 7 Minuten so aufbewahrt. Die Behandlung wurde durch eine 5-minütige Schlußwässerung mit 37°C warmem Wasser beendet und danach für 30 Minuten bei 45°C Umluft getrocknet.
Für eine drei- und sechsfache Dauerwellbehandlung wurde diese Prozedur in Zeitintervallen zwischen den einzelnen Behandlungen von 24 Stunden entsprechend oft wiederholt.

### Beispiel 3: Nichtbehandelte, bewetterte Haarproben

Ausgangsmaterial waren Haarzöpfe von unbehandelten, den normalen Wettereinflüssen ausgesetzte braune und blonde Haare verschiedener mitteleuropäischer Frauen. Um eine hinreichende Bewetterung sicherzustellen, wurden Haare mit einer Mindestlänge von 20 cm verwendet Die Haare wurden unmittelbar vor der entsprechenden proteolytischen oder hydrolytischen Behandlung vom Kopf entnommen. Verglichen wurden jeweils die Haaransätze (keine Bewetterung) mit den den Umweltbedingungen ausgesetzten Haarspitzen.

### Beispiel 4: Bestimmung des Trübungsgrades

Zur Bestimmung des Trübungsgrades wurde aus einem Ansatz mit proteoiytisch behandeltern Haar 1,0 ml aus dem Reaktionsansatz (Flüssigkeitsprobe) entnommen und mit dem Trübungsphotometer Typ Nephla LPG 23 (Dr. Bruno Lange GmbH, Berlin) inklusive Küvettenadapter und Glasküvette (11 mm), welches über eine dauerhafte Kalibrierkonstanz verfügt und dessen Genauigkeit im Kalibrierungspunkt von 10 FNU (TE/F) < 2% beträgt, vermessen. Die untersuchte Probe wurde daraufhin direkt in den Reaktionsansatz (Glasküvette, Meßzelle) für weitere Messungen zurückgegeben. Als Blindwert wurde 1,0 ml des Puffers (50 mM Natriumphosphatpuffer, pH 6,5) mit dem jeweiligen Reduktionsmittel (L-Cystein, DTT oder DTE) und dem jeweiligen Enzym (Papain, Pronase E oder Proteinase K) in Abwesenheit der Haare vermessen.
Mit dem Trübungsphotometer mit Küvette (Meßzelle) wurden Trübungsmessungen bis zu einer Temperatur von < 60°C (Temperatur des Reaktionsansatzes) durchgeführt. Aufgezeichnet wurde die Trübung nach Kalibration mit Formazin DIN-Standard wahlweise in FNU, TE/F oder mgll SiO₂, wobei dies auch für die modifizierte Vorrichtung gemäß Beispiel 5 galt.

### Beispiel 5: Vorrichtung für die direkte, kontinuierliche Messung der Trübung

Um den proteolytischen Abbau der Haarproben kontinuierlich und direkt (on-line) verfolgen zu können, wurde das Trübungsphotometer gemäß Beispiel 4 modifiziert, in dem eine Heizfolie mit Thermostatisierung und eine Rühreinheit integriert wurden. Hierzu wurde in die Wandung des Küvettenadapters für die 11 mm Rundküvetten eine Heizfolie (HR 5377R17.5L 12A, von Telemeter Electronic GmbH) und ein Thermoelement (RS 219-4719, von RS Components GmbH) integriert, die über einen Regler (CAL 3200, von CAL-Controls) angesteuert wurden. Die Rühreinheit bestand aus einem in die Küvette ragendem Rührer (Holzbohrer von 1,5 mm Durchmesser) und einem diesen Rührer antreibenden Elektromotor (SP 200 EC 3-5V DC, von SP. J. Schwarzer GmbH). Der Regler und der Elektromotor waren für Niederspannung ausgelegt und wurden von einem Transformator mit Spannung versorgt. In Fig. 1a und 1 b ist eine solche Vorrichtung schematisch abgebildet, Fig. 2 veranschaulicht eine alternative Vorrichtung.

### Beispiel 6: Standardisierung der Haarproben

Um für die Versuche zur proteolytischen Zersetzung standardisierte Ausgangsbedingungen betreffend die zu untersuchenden Haarproben zu schaffen, wurden die Haare vor der eigentlichen enzymatischen Behandlung standardmäßig für 2 Stunden mit 1% SDS gewaschen, anschließend sorgfältig mit destilliertem Wasser ausgewaschen und im Trockenschrank bei 37°C für 12 Stunden getrocknet.

### Beispiel 7: Proteolytische Zersetzung von einfach und dreifach oxidierten Haarproben mit Papain

Papain ist eine unspezifische Endopeptidase (Thiolprotease) aus *Carica papaya* mit Esterase- und Trans-Aminaseaktivität. Das Temperaturoptimum lag bei 50°C, das pH-Optimum zwischen 6,0 und 7,0 (EC 3.4.22.2). Das Enzym stammte von Boehringer Mannheim (Produkt-Nr: 1 693 379) und lag als Suspension vor (10 mg/ml).
Je 50,0 mg nicht oxidierter, einfach und dreifach oxidierter Haarproben (gemäß Beispiel 1) der Länge 1,0 cm wurden vor der Behandlung mit Papain in einem Gesamtvolumen von 5,0 ml Natriumphosphatpuffer (50 mM, pH 6,5) in einem verschließbaren Glasröhrchen für 1 Stunde bei 45°C suspendiert bzw. vorgequollen. Zur Aktivierung der Protease und Reduktion der Disulfitbrücken des Haares wurden L-Cystein (0,74 mg/ml), Natriummetabisulfit (Na₂S₂SO₃, 7,0 mg/ml) und Natriumsulfit (Na₂SO₃, 3,0 mg/ml) beigegeben. Nach Zugabe von 100 µl (21,6 U) einer Papain Suspension (10 mg/ml) wurde die Trübung mittels des Trübungsphotometers Typ Nephla LPG 23 (Beispiel 4) bestimmt. Die Reaktion wurde unter Rühren (Magnetrührer, 600 UpM) bei 45°C durchgeführt. Als Blindwert diente eine Haarprobe in Pufferlösung mit Reduktionsmitteln ohne Papain. Aufgetragen wurde jeweils eine Einfachbestimmung.
Die für dieses und für alle weiteren Beispiele verwendeten Reagenzien L-Cystein, Natriummetabisulfit, Natriumsulfit, stammten von Sigma (Deisenhofen).
Fig. 3 zeigt die erhaltenen Ergebnisse. Es ist deutlich zu erkennen, daß bei den zwei Oxidationsstufen, ein unterschiedlich starker Anstieg der Trübung eintritt. Bereits nach 40 Minuten waren alle Oxidationsstufen anhand ihres Trübungsverhaltens voneinander zu unterscheiden. Die Trübung des nicht oxidierten Haares nahm nur sehr leicht zu, während die Trübung des 3-fach oxidierten Haares nach 20 Minuten deutlich stärker anstieg als die der einfach oxidierten Haarprobe. Betreffend den Blindwert erfolgte überhaupt kein Anstieg einer Trübung. Es wird deutlich, daß das Trübungsverhalten vom Zustand der Haare bzw. dem Grad der Haarschädigung abhängt und damit korreliert.

### Beispiel 8: Proteolytische Zersetzung von bis zu 7-fach oxidierten Haarproben mit Papain

In Analogie zu Beispiel 7 wurden die Trübungen von Haarproben mit sieben verschiedenen Oxidationsstufen, wie in Beispiel 1 dargestellt, untersucht. In diesem Falle wurden kürzere Haarproben (je 50 mg) einer Länge von 0,5 cm gewählt, um die Reaktionsgeschwindigkeit zu erhöhen. Es wurden jeweils Einfachbestimmungen graphisch aufgetragen.
Fig. 4 zeigt das Ergebnis dieser Versuchsreihe. Es ließen sich die oxidativ verschieden vorbehandelten Haarproben nach 20 Minuten unterscheiden, wobei der Trübungsgrad mit der Oxidationsstufe zunahm. Es wird deutlich, daß das Trübungsverhalten vom Zustand der Haare bzw. dem Grad der Haarschädigung abhängt und damit korreliert.

### Beispiel 9: Proteolytische Zersetzung von einfach, zweifach und dreifach oxidierten Haarproben mit Pronase E

Pronase E ist ein unspezifisches Enzymgemisch aus Endo- und Exoproteasen aus *Streptomyces griseus.* Das Temperaturoptimum lag bei 35 - 40°C, das pH-Optimum zwischen 6,0 - 7,5 (EC 3.424.4k). Das von Sigma, Deisenhofen, verwendete Präparat wies eine spezifische Aktivität von 5,3 U/mg auf.
In Analogie zu Beispielen 7 und 8 wurden je 50,0 mg nicht oxidierter und einfach, zweifach und dreifach oxidierter Haarproben (gemäß Beispiel 1) der Länge 1,0 cm vor der Behandlung mit Pronase E in einem Gesamtvolumen von 5,0 ml Natriumphosphatpuffer (50 mM, pH 7,5) oder wahlweise Tris/HCI-Puffer (50 mM, pH 7,5) in einem verschließbaren Glasröhrchen für 1 Stunde bei 37°C suspendiert bzw. vorgequollen. Nach Zugabe von 2,0 mg (oder 4,0 mg) Pronase E (10,6 bzw. 21,2 U) wurden die Glasröhrchen bei 37°C unter Rühren (Magnetrührer, 600 UpM) im Wasserbad inkubiert und die Trübung mittels des Trübungsphotometers Typ Nephla LPG 23 (Beispiel 4) analog Beispielen 7 und 8 bestimmt. Auch hier wurden deutliche Unterschiede in den Trübungen in Abhängigkeit der verschiedenen Haarzustände (Haarschädigungen) erzielt.
Bei Wahl der höheren Konzentration von Pronase E (4,0 mg, entsprechend 21,2 U) wurde eine beschleunigte Zersetzung der Haarprobe erreicht.

### Beispiel 10: Proteolytische Zersetzung von einfach, zweifach und dreifach oxidierten Haarproben mit Proteinase K

Proteinase K ist eine aus *Tritirachium album* gewonnene Serin Endopeptidase, die die Peptidbindungen bevorzugt nach der Carboxyl-Gruppe von N-substituierten, hydrophoben, aliphatischen und aromatischen Aminosäuren hydrolysiert. Der optimale Temperaturbereich der verwendeten Proteinase K (Merck, Darmstadt) lag zwischen 25-35°C und der optimale pH-Wert zwischen 6,5-7,5 (EC 3.4.21.14d). Die Enzymaktivität für die suspendierte Proteinase K betrug 600 U/ml. Zur Aktivierung dieser Protease mußte zusätzlich Ca²⁺ zugegeben werden (BAJORATH, J. et al., Eur. J. Biochem. 176:441, 1988).
In Analogie zu den vorhergehenden Beispielen wurden je 50,0 mg nicht oxidierter und einfach, zweifach und dreifach oxidierter Haarproben (gemäß Beispiel 1) der Länge 0,5 cm vor der Behandlung mit Proteinase K in einem Gesamtvolumen von 5,0 ml 50 mM Tris/HCl mit 2 M Ca²⁺ (pH 7,5) in einem verschließbaren Glasröhrchen für 1 Stunde bei 25°C suspendiert bzw. vorgequollen. Nach Zugabe von 71 µl Proteinase K (42,2 U) wurden die Glasröhrchen bei 25°C unter Rühren (Magnetrührer, 600 UpM) im Wasserbad inkubiert und die Trübung mittels des Trübungsphotometers Typ Nephla LPG 23 (Beispiel 4) in Analogie zu den vorhergehenden Beispielen bestimmt. Auch hier wurden deutliche Unterschiede in den Trübungen in Abhängigkeit der verschiedenen Haarzustände (Haarschädigungen) erzielt.

### Beispiel 11: Proteolytische Zersetzung von einfach, dreifach und sechsfach dauergewellten Haarproben mit Papain

Analog zu Beispielen 7 bis 10 wurden verschieden stark dauergewellte Haare gemäß Beispiel 2 einem proteolytischen Verdau mit Papain ausgesetzt und die Zunahme der Trübung zur Beurteilung der Haarzustände bzw. Haarschädigungen herangezogen. Hierzu wurden 50 mg Haare einer Länge von 0,5 cm verwendet.
In Fig. 5 sind die Resultate graphisch dargestellt, wobei jeweils eine 1-fach Bestimmung aufgetragen wurde. Es sind deutliche Unterschiede in den Trübungen entsprechend den verschiedenen dauerwellbehandelten Haaren zu erkennen, welche mit den jeweiligen Haarzuständen (Haarschädigungen) korrelieren.
Es kann festgestellt werden, daß die durch Dauerwellbehandlung hervorgerufenen Schädigungen geringer waren, als die durch oxidative Behandlung hervorgerufenen Schäden.

### Beispiel 12: Vergleich der proteolytischen Zersetzung von bewetterten und unbewetterten, nicht behandelten Haarproben von dunkelbraunen und blonden Haaren

Zur Untersuchung von Bewetterungsschäden wurde das Trübungsverhalten von Haarproben aus Haaransatz und Haarspitze miteinander verglichen. Dabei wurde dunkelbraunes und blondes Haar verwendet, um den Einfluß der Haarfarbe bzw. der durch den enzymatischen Abbau gegebenenfalls freigesetzten Farbpigmente, bei dunkelbraunem Haar Eumelanin und bei blondem Haar Pheomelanin, auf die Trübung zu untersuchen. Diese Untersuchungen erfolgten in Analogie zu Beispiel 7 unter Verwendung von jeweils 50,0 mg Haarprobe (0,5 cm lang) aus Haaransatz und Haarspitze für jeweils beide Haarfarbtypen. Aufgetragen wurde jeweils eine einfache Bestimmung. Fig. 8a (dunkelbraune Haare) und Fig 8b (blonde Haare) veranschaulichen die erhaltenen Ergebnisse.
Aus den Kurvenverläufen ist zu erkennen, daß bewettertes Haar (Haarspitze) leichter proteolytisch abgebaut wurde und daher eine stärkere Trübung hervorrief, als unbewettertes Haar (Haaransatz). Eine Unterscheidung beider Haarsorten war bereits nach 10 Minuten möglich. Weiterhin zeigte sich, daß die unterschiedliche Haarfarbe und Haardicke keinen Einfluß auf die Trübungsmessung hatte.
Im Vergleich zu den Versuchen mit kosmetisch behandelten Haaren (oxidierte und dauergewellte Haare) war festzustellen, daß der proteolytische Abbau von bewettertem Haar langsamer voranschritt als von kosmetisch behandeltem Haar. Es kann daraus geschlossen werden, daß sich die Schädigungen durch Bewetterung hauptsächlich auf die Cuticula beschränken, während die kosmetische Behandlung auch strukturelle Veränderungen im Cortex verursacht (ROBBINS, C.R. & BAHL, M.J., J. Soc. Cosmet. Chem. 35:379, 1984).

### Beispiel 13: Vergleichende Untersuchungen verschiedener Haarproben

Es wurde ein Vergleich aller Trübungswerte durchgeführt, die anhand untersuchter Haarproben aus nicht behandelten (naturbewetterten), oxidierten und dauergewellten sowie dunkelbraunen und blonden Haaren erhalten wurden. Alle Trübungswerte wurden nach 30 Minuten Inkubation von 50 mg Haaren; 100 µl Papain (21,2 U); 5,0 ml 50 mM Natriumphosphatpuffer, pH 6,5; 0,74 mg/ml L-Cystein; 7,0 mg/ml Na₂S₂O₅; 3,0 mg/ml Na₂SO₃; 45°C; Rührgeschwindigkeit (Magnetrührer) 600 UpM, gemessen. Die Längen aller Haarproben betrugen 0,5 cm mit Ausnahme des Haartyps Nr. 6, hier betrug die Länge der Haarprobe 1,0 cm. Die Versuchs- und Reaktionsbedingungen entsprachen denen von Beispiel 7. Tabelle 2 zeigt die erhaltenen Trübungswerte von verschiedenen Haarproben des jeweiligen Haartyps. Haare, die vom Ansatz bzw. von der Spitze entnommen wurden, repräsentieren unbewettertes bzw. bewettertes Haar. Die Prozentangaben geben die jeweils verwendete H₂0₂ Konzentration bei der oxidativen Behandlung gemäß Beispiel 1 wieder.

**Tabelle 2**

| **Nr.** | **Haartyp** | **Trübung (TE/F)** |
|---|---|---|
| 1 | dunkelbrauner Haarzopf, unbehandelt (Haaransatz) | 20 |
| 2 | blonder Haarzopf, unbehandelt (Haaransatz) | 20 |
| 3 | dunkelbraune Kammsträhne, nicht oxidiert | 50 |
| 4 | dunkelbrauner Haarzopf (Haarspitze) | 60 |
| 5 | blonder Haarzopf (Haarspitze) | 60 |
| 6 | dunkelbraune Kammsträhne, 1-fach dauergewellt | 70 |
| 7 | dunkelbraune Kammsträhne, 2-fach dauergewellt | 120 |
| 8 | dunkelbraune Kammsträhne, 1-fach oxidiert (6%) | 130 |
| 9 | dunkelbraune Kammsträhne, 3-fach dauergewellt | 230 |
| 10 | dunkelbraune Kammsträhne, 2-fach oxidiert (6%) | 240 |
| 11 | dunkelbraune Kammsträhne, 3-fach oxidiert (6%) | 240 |
| 12 | dunkelbraune Kammsträhne, 4-fach oxidiert (6%) | 360 |
| 13 | dunkelbraune Kammsträhne, 5-fach oxidiert (6%) | 410 |
| 14 | dunkelbraune Kammsträhne, 3-fach oxidiert (9%) | 430 |
| 15 | dunkelbraune Kammsträhne, 6-fach oxidiert (6%) | 470 |
| 16 | dunkelbraune Kammsträhne, 7-fach oxidiert (6%) | 530 |

Die Haarproben vom Haaransatz der untersuchten Haarzöpfe wiesen den geringsten Schädigungsgrad auf. Die bewetterten dunkelbraunen und blonden Haarspitzen waren nur unwesentlich stärker geschädigt (TE/F = 60) als die dunkelbraune Kammsträhne des Haartyps Nr. 3 (TE/F = 50), die als Vergleich zu den behandelten Haaren diente. Die dauergewellten Haare zeigten im Vergleich zu den oxidierten Haaren eine geringere Schädigung. So entsprach das Haar der 3. Dauerwellstufe (Nr. 9) in der Schädigung den 2-fach oxidierten Haaren (Nr. 10). Deutlich war der Unterschied zwischen den stärker (9% H₂O₂) und schwächer gebleichten Haarproben (6% H₂O₂) der 3. Stufe (TE/F = 430 bzw. 240) zu sehen.

### Beispiel 14: Optimierung der proteolytischen Zersetzung

Es wurden, im Gegensatz zu den vorhergehenden Beispielen, die Haarproben auf eine Länge von 0,3 cm geschnitten und mit 500 µl (108 U) Papain versetzt. Verwendet wurden nicht-oxidierte, 1-fach und 3-fach oxidierte Haarproben. Die Oxidationen erfolgten gemäß Beispiel 1. Die Versuchs- und Reaktionsbedingungen entsprachen ansonsten denen von Beispiel 7. Fig. 6 veranschaulicht die erhaltenen Ergebnisse. Durch die Erhöhung des Papain Gehaltes auf 108 U pro Ansatz und der Reduzierung der Schnittlänge der Haare von 1,0 bzw. 0,5 cm auf 0,3 cm, konnte der proteolytische Abbau weiter beschleunigt werden, wodurch schon ab 10 Minuten eine Unterscheidung der Haarschädigungen gegeben war.

### Beispiel 15: Direkte, kontinuierliche Messung der Trübung bei Proteolyse von oxidierten Haarproben

Je 5,0 mg nicht oxidierte, 1-fach und 3-fach oxidierte Haare der Länge von 0,1 cm wurden in einem Gesamtvolumen von 3,0 ml 50 mM Natriumphosphatpuffer, pH 6,2, in einer 10 mm Glasküvette suspendiert. Zur Aktivierung der Protease und Reduktion der Disulfidbrücken des Haares wurden 10,0 mg/ml DTE (Sigma, Deisenhofen) zugegeben. Nach Erreichen der Reaktionstemperatur von 50°C wurden 100 µl (21,2 U) einer Papain Suspension (10 mg/ml) zugegeben und die Trübung direkt mittels des in Beispiel 5 beschriebenen modifizierten Photometers kontinuierlich bestimmt. Die Reaktion und die kontinuierliche Messung wurde unter Rühren (1mm Bohrer, 1000 UpM) durchgeführt. Fig. 7 zeigt den erhaltenen Kurvenverlauf der untersuchten Haarproben.
Nach bereits weniger als 10 Minuten war eine deutliche Unterscheidung zwischen den einzelnen Haarproben zu erkennen.

### Beispiel 16: Reproduzierbarkeit der Trübungsmessungen

Die Reproduzierbarkeit der Trübungsmessung wurde anhand von je 2 Ansätzen von nicht oxidierten und 3-fach oxidierten Haarproben (gemäß Beispiel 1) unter identischen Bedingungen überprüft. Hierzu wurde gemäß Beispiel 7 vorgegangen. Aufgetragen wurde jeweils der Mittelwert einer Doppelbestimmung. Als Ergebnis wurde festgestellt, daß der Fehler bei der Doppelbestimmung bei < 5% lag.

### Beispiel 17: Einfluß von Reduktionsmitteln auf das Trübungsverhalten bei der proteolytischen Zersetzung von Haarproben

Reduktionsmittel, wie beispielsweise Sulfite und L-Cystein, wurden zur Aktivierung der Protease Papain verwendet. Sulfite und L-Cystein dringen dabei durch Diffusion ins Haar ein und reduzieren Disulfitbrücken. In Gegenwart von L-Cystein und Sulfiten kommt es zur Bildung des sogenannten "Bunte Salzes" (CLARK, H.T., J. Biol. Chem.97:235, 1932). Es wurde überprüft, ob diese Veränderung in der Tertiärstruktur den proteolytischen Abbau der Haare zu beeinflussen bzw. zu beschleunigen vermag. Hierzu wurde zu je einem Ansatz nicht oxidiertem und 3-fach oxidiertem Haar (je 50,0 mg, 0,5 cm lang) die in den Beispielen verwendeten Sulfitmengen (7,0 mglml Natriummetabisulfit, Na₂S₂SO₃, 3,0 mg/ml Natriumsulfit, Na₂SO₃) zusammen mit L-Cystein (0,74 mg/ml) zugegeben (Lösung A). Alternativ wurde der Sulfitgehalt durch alleinige Zugabe von L-Cystein in der Konzentration 10,68 mg/ml ersetzt (Lösung B). Das Reaktionsvolumen (Flotte), die Mengen an Papain und Natriumphosphatpuffer (pH 6,5), die Temperatur und die Rührgeschwindigkeit entsprachen Beispiel 7.
Es zeigte sich, daß sich die Ansätze mit (Lösung A) und ohne Sulfite (Lösung B) nur unwesentlich unterschieden.

### Beispiele 18 - 77: Verschiedene enzymatisch/chemische Behandlungen von Haarproben mit Papain

Die proteolytische Zersetzung von Haarproben gemäß nachfolgender Beispiele erfolgte in einem Gesamtvolumen (Flotte) von 3,0 ml. Die Haarbehandlungen (Oxidationen, Dauerwellbehandlungen, Bewetterungen) wurden gemäß Beispielen 1, 2 und 3 durchgeführt. Die Zersetzungen erfolgten nach dem Verfahren gemäß Beispiel 6 und Beispiel 7.

| **Beispiel** | **Haarprobe** | **Haarbehandlung** | **Temp.** | **pH** | **Papain (U)** | **Reduktionsmittel** | **Bemerkung** |
|---|---|---|---|---|---|---|---|
| **18** | 2,0 mg, 1,0 mm | 3-fach oxidiert | 45°C | 6,5 | 21,6 | 0,74 mg/ml Cys, 7 mg/ml Na2S2O5, 3 mg Na2SO3 | V⁽¹⁾ |
| **19** | 5,0 mg, 1,0 mm | 3-fach oxidiert | 45°C | 6,5 | 21,6 | 0,74 mg/ml Cys, 7 mg/ml Na2S2O5, 3 mg Na2SO3 | V |
| **20** | 5,0 mg, 1.0mm | 3-fach oxidiert | 45°C | 6,5 | 21,6 | 10 mg/ml DTT | V |
| **21** | 5,0 mg, 1,0 mm | nicht oxidiert | 45°C | 6,5 | 21,6 | 10 mg/ml DTT | V |
| **22** | 5,0 mg, 1,0 mm (W)⁽²⁾ | 3-fach oxidiert | 45°C | 6,5 | 21,6 | 10 mg/ml DTT | V |
| **23** | 5,0 mg, 1,0 mm (W) | 3-fach oxidiert | 45°C | 8,0 | 21,6 | 10 mg/ml DTE | V |
| **24** | 20 mg, 1,0 mm (W) | 3-fach oxidiert | 45°C | 8,0 | 21,6 | 10 mg/ml DTE | V |
| **25** | 10 mg, 1,0 mm (W) | 3-fach oxidiert | 45°C | 6,5 | 21,6 | 10 mg/ml DTE | V |
| **26** | 5,0 mg, 1,0 mm (W) | 3-fach oxidiert | 45°C | 6,5 | 21,6 | 10 mg/ml DTE | V |
| **27** | 5,0 mg, 1,0 mm (W) | 3-fach oxidiert | 45°C | 7,0 | 21,6 | 10 mg/ml DTE | V |
| **28** | 5,0 mg, 1,0 mm (W) | 3-fach oxidiert | 45°C | 7,0 | 21,6 | 10 mg/ml DTT | V |
| **29** | 5,0 mg, 1,0 mm (W) | 3-fach oxidiert | 45°C | 6,0 | 21,6 | 10 mg/ml DTE | V |
| **30** | 5,0 mg, 1,0 mm (W) | 3-fach oxidiert | 45°C | 6,2 | 21,6 | 10 mg/ml DTE | V |
| **31** | 5,0 mg, 1,0 mm (W) | 3-fach oxidiert | 45°C | 6,2 | 43,2 | 10 mg/ml DTE | V |
| **32** | 5,0 mg, 1,0 mm (W) | 3-fach oxidiert | 50°C | 6,2 | 21,6 | 10 mg/ml DTE | V |
| **33** | 5,0 mg, 23,0 mm (W) | 3-fach oxidiert | 50°C | 6,2 | 21,6 | 10 mg/ml DTE | V |
| **34** | 15 Haare (2,0 mg) 46,0 mm (W) | 3-fach oxidiert | 50°C | 6,2 | 21,6 | 10 mg/ml DTE | V |
| **35** | 15 Haare (2,0 mg) 46,0 mm (W) | nicht oxidiert | 50°C | 6,2 | 21,6 | 10 mg/ml DTE | V |
| **36** | 100 Haare (14 mg) 46,0 mm (W) | 3-fach oxidiert | 50°C | 6,2 | 21,6 | 10 mg/ml DTE | V |
| **37** | 100 Haare (14 mg) 46,0 mm (W) | nicht oxidiert | 50°C | 6,2 | 21,6 | 10 mg/ml DTE | V |
| **38** | 5,0 mg, 10,0 mm (W) | 3-fach oxidiert | 50°C | 6,2 | 21,6 | 10 mg/ml DTE | V |
| **39** | 5,0 mg, 1,0 mm (W) | 1-fach oxidiert | 50°C | 6,2 | 21,6 | 10 mg/ml DTE | V |
| **40** | 5,0 mg, 1,0 mm (W) | 2-fach oxidiert | 50°C | 6,2 | 21,6 | 10 mg/ml DTE | V |
| **41** | 5,0 mg, 1,0 mm (W) | 3-fach oxidiert | 50°C | 6,2 | 10,8 | 10 mg/ml DTE | V |
| **42** | 5,0 mg, 1,0 mm | bewettert, Haaransatz | 50°C | 6,2 | 21,6 | 10 mg/ml DTE | V |
| **43** | 5,0 mg, 1,0 mm | bewettert, Haarspitze | 50°C | 6,2 | 21,6 | 10 mg/ml DTE | V |
| **44** | 5,0 mg, 1,0 mm (W) | 3-fach oxidiert | 50°C | 7,0 | 21,6 | 20 mg/ml Cystein | V |
| **45** | 5,0 mg, 1,0 mm (W) | 3-fach oxidiert | 50°C | 7,5 | 21,6 | 20 mg/ml Cystein | V |
| **46** | 5,0 mg, 1,0 mm (W) | 3-fach oxidiert | 50°C | 6,5 | 21,6 | 20 mg/ml Cystein | V |
| **47** | 5,0 mg, 1,0 mm (W) | nicht oxidiert | 50°C | 6,5 | 21,6 | 20 mg/ml Cystein | V |
| **48** | 5,0 mg, 1,0 mm (W) | 1-fach oxidiert | 50°C | 6,5 | 21,6 | 20 mg/ml Cystein | V |
| **49** | 5,0 mg, 1,0 mm (W) | 2-fach oxidiert | 50°C | 6,5 | 21,6 | 20 mg/ml Cystein | V |
| **50** | 5,0 mg, 1,0 mm (W) | 3-fach oxidiert | 50°C | 6,5 | 21,6 | 20 mg/ml Cystein | V |
| **51** | 5,0 mg, 1,0 mm (W) | nicht oxidiert | 50°C | 5,5 | 21,6 | 20 mg/ml Cystein | V |
| **52** | 5,0 mg, 1,0 mm (W) | 1-fach oxidiert | 50°C | 5,5 | 21,6 | 20 mg/ml Cystein | V |
| **53** | 5,0 mg, 1,0 mm (W) | 3-fach oxidiert | 50°C | 6,5 | 21,6 | 20 mg/ml Cystein | V |
| **54** | 5,0 mg, 1,0 mm (W) | nicht oxidiert | 35°C | 6,5 | 21,6 | 20 mg/ml Cystein | V |
| **55** | 5,0 mg, 1,0 mm (W) | 1-fach oxidiert | 35°C | 6,5 | 21,6 | 20 mg/ml Cystein | V |
| **56** | 5,0 mg, 1,0 mm (W) | 3-fach oxidiert | 35°C | 6,5 | 21,6 | 20 mg/ml Cystein | V |
| **57** | 5,0 mg, 1,0 mm (W) | 3-fach oxidiert | 50°C | 6,5 | 21,6 | 2,5 ml Phosphat pH 6,5; 0,5 ml 120 mg/ml Cystein | S⁽³⁾ |
| **58** | 5,0 mg, 1,0 mm (W) | 1-fach oxidiert | 50°C | 6,5 | 21,6 | 2,5 ml Phosphat pH 6,5; 0,5 ml 120 mg/ml Cystein | S |
| **59** | 5,0 mg, 1,0 mm (W) | nicht oxidiert | 50°C | 6,5 | 21,6 | 2,5 ml Phosphat pH 6,5; 0,5 ml 120 mg/ml Cystein | S |
| **60** | 5,0 mg, 1,0 mm (W) | 1-fach oxidiert | 40°C | 6,5 | 21,6 | 20 mg/ml Cystein | V |
| **61** | 5,0 mg, 1,0 mm (W) | 3-fach oxidiert | 40°C | 6,5 | 21,6 | 20 mg/ml Cystein | V |
| **62** | 5,0 mg, 1,0 mm (W) | 1-fach oxidiert | 40°C | 6,5 | 21,6 | 20 mg/ml Cystein + 20% EtOH | V |
| **63** | 5,0 mg, 1,0 mm (W) | 3-fach oxidiert | 40°C | 6,5 | 21,6 | 20 mg/ml Cystein + 20% EtOH | V |
| **64** | 5,0 mg, 1,0 mm (W) | 1-fach oxidiert | 40°C | 6,5 | 21,6 | 20 mg/ml Cystein + 10% EtOH | V |
| **65** | 5,0 mg, 1,0 mm (W) | 3-fach oxidiert | 40°C | 6,5 | 21,6 | 20 mg/ml Cystein + 10% EtOH | V |
| **66** | 5,0 mg, 1,0 mm (W) | 1-fach oxidiert | 40°C | 6,5 | 21,6 | 10 mg/ml Cystein + 20% EtOH | V |
| **67** | 5,0 mg, 1,0 mm (W) | 3-fach oxidiert | 40°C | 6,5 | 21,6 | 10 mg/ml Cystein + 20% EtOH | V |
| **68** | 5,0 mg, 1,0 mm (W) | 3-fach oxidiert | 40°C | 6,5 | 21,6 | 0,74 mg/ml Cystein 7,0 mg/ml Na₂S₂O₅ 3,0 mg/ml Na₂SO₃ | V |
| **69** | 5,0 mg, 1,0 mm (W) | 3-fach oxidiert | 40°C | 6,5 | 21,6 | 10,74 mg/ml Cystein | V |
| **70** | 5,0 mg, 1,0 mm (W) | 3-fach oxidiert | 40°C | 6,5 | 21,6 | 10 mg/ml Cystein 7,0 mg/ml Na₂S₂O₅ 3,0 mg/ml Na₂SO₃ | V |
| **71** | 5,0 mg, 1,0 mm (W) | nicht oxidiert | 50°C | 6,5 | 21,6 | 10 mg/ml DTE | V |
| **72** | 5,0 mg, 1,0 mm | bewettert, Haarspitze | 50°C | 6,5 | 21,6 | 10 mg/ml DTE | V |
| **73** | 5,0 mg, 1,0 mm | bewettert, Haaransatz | 50°C | 7,0 | 21,6 | 10 mg/ml DTE | V |
| **74** | 5,0 mg, 1,0 mm | bewettert, Haaransatz | 50°C | 6,5 | 21,6 | 10 mg/ml DTE | V |
| **75** | 5,0 mg, (W) 1,0 mm | 6 x DW | 50°C | 6,5 | 21,6 | 20 mg/ml Cystein | V |
| **76** | 5,0 mg, (W) 1,0 mm | 3 x DW | 50°C | 6,5 | 21,6 | 20 mg/ml Cystein | V |
| **77** | 5,0 mg, (W) 1,0 mm | 1 x DW | 50°C | 6,5 | 21,6 | 20 mg/ml Cystein | V |

| | | | | | | | |
|---|---|---|---|---|---|---|---|
| ⁽¹⁾ V = 5 Minuten Vorinkubation, dann Papain, | | | | | | | |
| ⁽²⁾ (W) = Haare mit 1% SDS gemäß Beispiel 6 gewaschen, | | | | | | | |
| ⁽³⁾ S = 5 Minuten Vorinkubation, dann simultane Gabe von Cystein und Papain, ⁽⁴⁾ DW = Dauerwellbehandlung gemäß Beispiel 2 | | | | | | | |

## Patentansprüche

1. Verfahren zur Feststellung und Bestimmung des Zustandes von Keratin enthaltenden Hautanhangsgebilden, **dadurch gekennzeichnet, daß** eine Probe Keratin enthaltende Hautanhangsgebilde in einer wäßrigen Lösung einer enzymatischen und/oder chemischen Behandlung unterworfen wird, und anschließend der Zustand der Keratin enthaltenden Hautanhangsgebilde anhand der Trübung der erhaltenen Flüssigkeitsprobe untersucht, ausgewertet und bestimmt wird.

2. Verfahren nach Anspruch 1, **dadurch gekennzeichnet, daß** die Untersuchung der Flüssigkeitsprobe optisch oder visuell erfolgt.

3. Verfahren nach Anspruch 2, **dadurch gekennzeichnet, daß** die Untersuchung der Flüssigkeitsprobe mittels physikalischer Meßmethoden erfolgt.

4. Verfahren nach Anspruch 3, **dadurch gekennzeichnet, daß** die physikalischen Meßmethoden auf Basis von Lichtstreuungsmessungen, nephelometrischen Messungen, Fluoreszenzstrahlungsmessungen, Durchlichtmessungen bzw. Extinktionsmessungen, turbidimetrischen Messungen (Turbidimetrie) erfolgen.

5. Verfahren nach einem der Ansprüche 1 bis 4, **dadurch gekennzeichnet, daß** die Untersuchung und Auswertung der Flüssigkeitsprobe durch Trübungsmessung erfolgt.

6. Verfahren nach Anspruch 5, **dadurch gekennzeichnet, daß** die Trübungsmessung mittels eines Photometers erfolgt.

7. Verfahren nach Ansprüchen 1 bis 6, **dadurch gekennzeichnet, daß** die enzymatische und/oder chemische Behandlung der Probe an Keratin enthaltenden Hautanhangsgebilden auf Basis einer proteolytischen oder hydrolytischen Reaktion erfolgt.

8. Verfahren nach Anspruch 7, **dadurch gekennzeichnet, daß** die proteolytische oder hydrolytische Reaktion mit Hilfe mindestens eines Enzyms, einzeln oder als Gemisch, erfolgt, aus der Gruppe der Proteasen und Proteinasen, die in der Lage sind, einen Abbau von keratinhaltigem Material zu katalysieren.

9. Verfahren nach Anspruch 8, **dadurch gekennzeichnet, daß** die Proteasen und Proteinasen ausgewählt sind aus Papain, Pronase E, Proteinnase K, Subtilisin Trypsin oder Keratinasen.

10. Verfahren nach Anspruch 7, **dadurch gekennzeichnet, daß** die proteolytische oder hydrolytische Reaktion mit Hilfe von mindestens einem keratolytisch wirksamen und/oder reduzierenden organischen und/oder anorganischen Reagenz erfolgt.

11. Verfahren nach Anspruch 10, **dadurch gekennzeichnet, daß** das organische und/oder anorganische Reagenz mindestens eine der Verbindungen Harnstoff, Natriumhydroxid, Tributylphosphin, Natriumjodid, Perameisensäure, Ammoniak, Thioglykolsäure, Essigsäure, Cystein, Natriummetabisulfit, Natriumsulfit, Dithiothreitol oder Dithioerythrol umfaßt.

12. Verfahren nach Ansprüchen 7 bis 11, **dadurch gekennzeichnet, daß** die proteolytische und/oder hydrolytische Reaktion mit Hilfe mindestens eines Enzyms in Kombination mit mindestens einem keratolytisch wirksamen und/oder reduzierenden organischen und/oder anorganischen Reagenz erfolgt.

13. Verfahren nach Ansprüchen 1 bis 12, **dadurch gekennzeichnet, daß** nicht durch die enzymatische und/oder chemische Behandlung zersetzte Bestandteile der Keratin enthaltenden Hautanhangsgebilde vor der Untersuchung der Flüssigkeitsprobe entfernt werden oder während der Untersuchung entfernt gehalten werden, und daß die Untersuchung und Auswertung der Flüssigkeitsprobe in Abwesenheit nicht zersetzter Bestandteile der Keratin enthaltenden Hautanhangsgebilde durchgeführt wird.

14. Verfahren nach einem der Ansprüche 1 bis 13, **dadurch gekennzeichnet, daß** die Probe Keratin enthaltende Hautanhangsgebilde während der enzymatischen und/oder chemischen Behandlung einer zusätzlichen mechanischen Belastung unterworfen wird.

15. Verfahren nach Ansprüchen 1 bis 14, **dadurch gekennzeichnet**, das die Untersuchung und Auswertung der Flüssigkeitsprobe während und/oder nach der enzymatischen und/oder chemischen Behandlung der Probe Keratin enthaltender Hautanhangsgebilde erfolgt.

16. Verfahren nach Anspruch 15, **dadurch gekennzeichnet**, das die Untersuchung und Auswertung der Flüssigkeitsprobe indirekt und diskontinuierlich oder direkt und kontinuierlich (on-line) erfolgt.

17. Verfahren nach Ansprüchen 1 bis 16, **dadurch gekennzeichnet, daß** die Untersuchung und Auswertung der Flüssigkeitsprobe unter Zuhilfenahme eines Computers und/oder eines Schreibers und/oder eines Bildschirms (Display) und/oder eines Druckers erfolgt.

18. Verfahren nach Ansprüchen 1 bis 17, **dadurch gekennzeichnet, dass** die Hautanhangsgebilde Haare sind.

19. Verwendung eines Trübungsphotometers zur Festellung und Bestimmung des Zustandes von Keratin enthaltenden Hautanhangsgebilden in einem der Verfahren gemäß Ansprüchen 1 bis 18.

20. Verwendung einer Vorrichtung bestehend aus einem Photometer mit Reaktionsgefäß und Adapter oder Halterung dafür in Kombination mit einer Heizung zur Erwärmung des Reaktionsgefäßes, einer Vermischvorrichtung für die Vermischung der Flüssigkeitsprobe im Reaktionsgefäß und einem steuerbaren Antrieb für die Vermischvorrichtung zur Festellung und Bestimmung des Zustandes von Keratin enthaltenden Hautanhangsgebilden in einem der Verfahren gemäß Ansprüchen 1 bis 18.

21. Verwendung einer Vorrichtung nach Anspruch 20, **dadurch gekennzeichnet, dass** die Heizung mit einem Thermoelement verbunden ist.

22. Verwendung einer Vorrichtung nach Anspruch 21, **dadurch gekennzeichnet, dass** die Heizung als Heizfolie vorliegt.

23. Verwendung einer Vorrichtung nach Anspruch 22, **dadurch gekennzeichnet, dass** die Heizfolie in der Wandung des Adapters oder Halters des Reaktionsgefäßes integriert ist.

24. Verwendung einer Vorrichtung nach Anspruch 23, **dadurch gekennzeichnet, dass** das Reaktionsgefäß ein Reagenzröhrchen oder eine Küvette ist.

25. Verwendung einer Vorrichtung nach Anspruch 24, **dadurch gekennzeichnet, dass** die Vermischvorrichtung aus einer Spritze mit Stempel oder Kolben oder aus einem Rührer besteht, welche über einen mechanischen, elektrischen oder magnetischen Antrieb angetrieben oder bewegt werden, oder aus einem Ultraschall erzeugenden Gerät.

26. Verwendung einer Vorrichtung nach Anspruch 25, **dadurch gekennzeichnet, dass** die Vorrichtung funktionell mit einem Schreiber und/oder Drucker und/oder Bildschirm (Display) und/oder Rechner (Computer) verbunden ist.

27. Verwendung einer Vorrichtung nach Anspruch 26, **dadurch gekennzeichnet, dass** der Rechner in Form eines Handgerätes ausgestaltet ist und mobil benutzt werden kann.

28. Verwendung eines Kits umfassend eine Füfferlösung, mindestens ein Enzym und/oder mindestens ein proteolytisch oder hydrolytisch wirkendes und keratinolytisch wirksames und/oder reduzierendes chemisches Reagenz zur Festellung und Bestimmung des Zustandes von Keratin enthaltenden Hautanhangsgebilden in einem der Verfahren gemäß Ansprüchen 1 bis 18.

29. Verwendung eines Kits nach Anspruch 28, **dadurch gekennzeichnet, dass** dieser zusätzlich eine Probe aus Keratin enthaltenden Hautanhangsgebilden und/oder eine Flüssigkeitsprobe als Standard-, Blind- oder Nullwert enthält.

30. Verwendung eines Kits nach Ansprüchen 28 und 29, **dadurch gekennzeichnet, dass** der Kit zusätzlich eine Vorrichtung aufweist wie definiert in Ansprüchen 20 bis 27.

## Claims

1. Method for ascertaining and determining the state of skin appendages containing keratin, **characterized in that** a sample of keratin-containing skin appendage in an aqueous solution is subjected to enzymatic and/or chemical treatment, and then the state of the keratin-containing skin appendage is analysed, evaluated and determined by reference to the opacity of the resulting liquid sample.

2. Method according to Claim 1, **characterized in that** the liquid sample is analysed optically or visually.

3. The method according to Claim 2, **characterized in that** the liquid sample is analysed by means of physical measurement methods

4. The method according to Claim 3, **characterized in that** the physical measurement methods take place on the basis of light-scattering measurements, nephelometric measurements, fluorescent radiation measurements, transmitted-light measurements or absorbance measurements, turbidimetric measurements (turbidimetry).

5. Method according to one of Claims 1 to 4, **characterized in that** the liquid sample is analysed and evaluated by measuring the turbidity.

6. Method according to Claim 5, **characterized in that** the turbidity is measured using a photometer.

7. Method according to Claims 1 to 6, **characterized in that** the enzymatic and/or chemical treatment of the sample of keratin-containing skin appendages is carried out on the basis of a proteolytic or hydrolytic reaction.

8. Method according to Claim 7, **characterized in that** the proteolytic or hydrolytic reaction takes place with the help of at least one enzyme, individually or as a mixture, from the group of proteases and proteinases which are able to catalyse a degradation of keratin-containing substance.

9. Method according to Claim 8, **characterized in that** the proteases and proteinases are chosen from papain, pronase E, proteinase K, subtilisin, trypsin or keratinases.

10. Method according to Claim 7, **characterized in that** the proteolytic or hydrolytic reaction is carried out with the help of at least one keratolytically effective and/or reductive organic and/or inorganic reagent.

11. Method according to Claim 10, **characterized in that** the organic and/or inorganic reagent comprises at least one of the compounds urea, sodium hydroxide, tributylphosphine, sodium iodide, performic acid, ammonia, thioglycolic acid, acetic acid, cysteine, sodium metabisulphite, sodium sulphite, dithiothreitol or dithioerythrol.

12. Method according to Claims 7 to 11, **characterized in that** the proteolytic and/or hydrolytic reaction is carried out with the help of at least one enzyme in combination with at least one keratolytically effective and/or reductive organic and/or inorganic reagent.

13. Method according to Claims 1 to 12, **characterized in that** constituents of the keratin-containing skin appendage not, decomposed by the enzymatic and/or chemical treatment are removed prior to analysing the liquid sample or are kept at a distance during the analysis, and **in that** the analysis and evaluation of the liquid sample is carried out in the absence of undecomposed constituents of the keratin-containing skin appendage.

14. Method according to one of Claims 1 to 13, **characterized in that** the sample of keratin-containing skin appendage is subjected to an additional mechanical strain during the enzymatic and/or chemical treatment.

15. Method according to Claims 1 to 14, **characterized in that** the liquid sample is analysed and evaluated during and/or after the enzymatic and/or chemical treatment of the sample of keratin-containing skin appendage.

16. Method according to Claim 15, **characterized in that** the analysis and evaluation of the liquid sample takes place indirectly and discontinuously or directly and continuously (on-line).

17. Method according to Claims 1 to 16, **characterized in that** the liquid sample is analysed and evaluated with the help of a computer and/or a recorder and/or a screen (display) and/or a printer.

18. Method according to Claims 1 to 17, **characterized in that** the skin appendages are hair.

19. Use of a turbidity photometer for ascertaining and determining the state of keratin-containing skin appendages in one of the methods according to Claims 1 to 18.

20. Use of a device consisting of a photometer with reaction vessel and adapter or holder therefor in combination with heating for warming the reaction vessel, a mixing device for mixing the liquid sample in the reaction vessel and a controllable drive for the mixing device for ascertaining and determining the state of keratin-containing skin appendages in one of the methods according to Claims 1 to 18.

21. Use of a device according to Claim 20, **characterized in that** the heating is connected to a thermocouple.

22. Use of a device according to Claim 21, **characterized in that** the heating is in the form of a heating foil.

23. Use of a device according to Claim 22, **characterized in that** the heating foil is integrated into the wall of the adapter or holder of the reaction vessel.

24. Use of a device according to Claim 23, **characterized in that** the reaction vessel is a test tube or a cuvette.

25. Use of a device according to Claim 24, **characterized in that** the mixing device consists of a syringe with punch or plunger or of a stirrer which are driven or agitated by means of a mechanical, electric or magnetic drive, or of an instrument producing ultrasound.

26. Use of a device according to Claim 25, **characterized in that** the device is functionally connected to a recorder and/or printer and/or screen (display) and/or computer.

27. Use of a device according to Claim 26, **characterized in that** the computer is designed in the form of a hand-held device and can be used on the move.

28. Use of a kit comprising a buffer solution, at least one enzyme and/or at least one proteolytically or hydrolytically effective and keratino-lytically effective and/or reductive chemical reagent for ascertaining and determining the state of keratin-containing skin appendages in one of the methods according to Claims 1 to 18.

29. Use of a kit according to Claim 28, **characterized in that** this additionally comprises a sample of keratin-containing skin appendages and/or a liquid sample as standard, blank or zero value.

30. Use of a kit according to Claims 28 and 29, **characterized in that** the kit additionally has a device as defined in Claims 20 to 27.

## Revendications

1. Procédé pour l'examen et la détermination de l'état d'annexes de la peau contenant de la kératine, **caractérisé en ce qu'**on soumet à un traitement chimique et/ou enzymatique un échantillon d'annexe de la peau contenant de la kératine, dans une solution aqueuse, et ensuite on analyse, évalue et détermine l'état de l'annexe de la peau contenant de la kératine à l'aide du trouble de l'échantillon liquide obtenu.

2. Procédé selon la revendication 1, **caractérisé en ce que** l'analyse de l'échantillon liquide s'effectue visuellement ou optiquement.

3. Procédé selon la revendication 2, **caractérisé en ce que** l'analyse de l'échantillon liquide s'effectue par des méthodes physiques de mesure.

4. Procédé selon la revendication 3, **caractérisé en ce que** les méthodes physiques de mesure s'effectuent à base de mesures de la dispersion de la lumière, de mesures néphélométriques, de mesures de rayonnement fluorescent, de mesures de lumière transmise ou de mesures d'extinction, de mesures turbidimétriques (turbidimétrie).

5. Procédé selon l'une quelconque des revendications 1 à 4, **caractérisé en ce que** l'analyse et l'évaluation de l'échantillon liquide s'effectuent par mesure du trouble.

6. Procédé selon la revendication 5, **caractérisé en ce que** la mesure du trouble s'effectue au moyen d'un photomètre.

7. Procédé selon les revendications 1 à 6, **caractérisé en ce que** le traitement chimique et/ou enzymatique de l'échantillon d'annexes de la peau contenant de la kératine s'effectue à base d'une réaction protéolytique ou hydrolytique.

8. Procédé selon la revendication 7, **caractérisé en ce que** la réaction protéolytique ou hydrolytique s'effectue à l'aide d'au moins une enzyme, seule ou en mélange, choisie dans le groupe des protéases et protéinases qui sont capables de catalyser une dégradation d'une matière contenant de la kératine.

9. Procédé selon la revendication 8, **caractérisé en ce que** les protéases et protéinases sont choisies parmi la papaïne, la pronase E, la protéinase K, la protéinase K, la subtilisine, la trypsine et les kératinases.

10. Procédé selon la revendication 7, **caractérisé en ce que** la réaction protéolytique ou hydrolytique s'effectue à l'aide d'au moins un réactif organique et/ou minéral réducteur et/ou à activité kératolytique.

11. Procédé selon la revendication 10, **caractérisé en ce que** le réactif organique et/ou minéral comprend au moins un des composés urée, hydroxyde de sodium, tributylphosphine, iodure de sodium, acide peformique, ammoniac, acide thioglycolique, acide acétique, cystéine, métabisulfite de sodium, sulfite de sodium, dithiothréitol ou dithioérythrol.

12. Procédé selon les revendications 7 à 11, **caractérisé en ce que** la réaction protéolytique et/ou hydrolytique s'effectue à l'aide d'au moins une enzyme en association avec au moins un réactif organique et/ou minéral réducteur et/ou à activité kératolytique.

13. Procédé selon les revendications 1 à 12, **caractérisé en ce que** les composants des annexes de la peau, contenant de la kératine, non détruits par le traitement chimique et/ou enzymatique, sont éliminés avant l'analyse de l'échantillon liquide ou maintenus séparés pendant l'analyse, et **en ce que** l'analyse et l'évaluation de l'échantillon liquide sont effectuées en absence des composants non détruits de l'annexe de peau contenant de la kératine.

14. Procédé selon l'une quelconque des revendications 1 à 13, **caractérisé en ce que** l'échantillon d'annexe de peau contenant de la kératine est soumis à une contrainte mécanique supplémentaire pendant le traitement chimique et/ou enzymatique.

15. Procédé selon les revendications 1 à 14, **caractérisé en ce que** l'analyse et l'évaluation de l'échantillon liquide s'effectuent pendant et/ou après le traitement chimique et/ou enzymatique de l'échantillon d'annexe de peau contenant de la kératine.

16. Procédé selon la revendication 15, **caractérisé en ce que** l'analyse et l'évaluation de l'échantillon liquide s'effectuent indirectement et en mode discontinu ou directement et en continu (en ligne).

17. Procédé selon les revendications 1 à 16, **caractérisé en ce que** l'analyse et l'évaluation de l'échantillon liquide s'effectuent à l'aide d'un ordinateur et/ou d'un enregistreur et/ou d'un écran (afficheur) et/ou d'une imprimante.

18. Procédé selon les revendications 1 à 17, **caractérisé en ce que** les annexes de la peau sont des cheveux.

19. Utilisation d'un turbophotomètre pour l'examen et la détermination de l'état d'annexes de la peau contenant de la kératine, dans un procédé selon les revendications 1 à 18.

20. Utilisation d'un dispositif constitué d'un photomètre avec récipient de réaction et adaptateur ou support pour ceux-ci en association avec un chauffage pour le chauffage du récipient de réaction, un dispositif de mélange pour le mélange de l'échantillon liquide dans le récipient de réaction et d'un mécanisme d'entraînement pouvant être commandé pour le dispositif de mélange, pour l'examen et la détermination de l'état d'annexes de la peau contenant de la kératine, dans l'un des procédés selon les revendications 1 à 18.

21. Utilisation d'un dispositif selon la revendication 20, **caractérisée en ce que** le chauffage est relié à un thermo-élément.

22. Utilisation d'un dispositif selon la revendication 21, **caractérisée en ce que** le chauffage est présent sous forme d'un film chauffant.

23. Utilisation d'un dispositif selon la revendication 22, **caractérisée en ce que** le film chauffant est intégré dans la paroi de l'adaptateur ou du support du récipient de réaction.

24. Utilisation d'un dispositif selon la revendication 23, **caractérisée en ce que** le récipient de réaction est un petit tube à essai ou une cuve.

25. Utilisation d'un dispositif selon la revendication 24, **caractérisée en ce que** le dispositif de mélange est constitué d'une seringue avec piston ou mandrin ou d'un agitateur, qui sont actionnés ou mus par un entraînement mécanique, électrique ou magnétique, ou d'un appareil produisant des ultrasons.

26. Utilisation d'un dispositif selon la revendication 25, **caractérisée en ce que** le dispositif est fonctionnement relié à un enregistreur et/ou une imprimante et/ou un écran (afficheur) et/ou un ordinateur (calculateur).

27. Utilisation d'un dispositif selon la revendication 26, **caractérisée en ce que** l'ordinateur est sous forme d'un appareil portable et peut être utilisé de façon mobile.

28. Utilisation d'un nécessaire comprenant une solution tampon, au moins une enzyme et/ou au moins un réactif chimique réducteur et/ou à action protéolytique ou hydrolytique et à activité keratinolytique, pour l'examen et la détermination de l'état d'annexes de la peau contenant de la kératine, dans un des procédés selon les revendications 1 à 18.

29. Utilisation d'un nécessaire selon la revendication 28, **caractérisée en ce que** celui-ci comporte en outre un échantillon d'annexes de peau contenant de la kératine et/ou un échantillon liquide en tant que valeur de référence, valeur à blanc ou zéro.

30. Utilisation d'un nécessaire selon les revendications 28 et 29, **caractérisée en ce que** le nécessaire comporte additionnellement un dispositif tel que défini dans les revendications 20 à 27.
